# EUROPEAN PATENT SPECIFICATION

(11) **EP 4 297 855 B1**
(45) Date of publication and mention of the grant of the patent: **16.09.2026**
(21) Application number: 22709941.3
(22) Date of filing: 24.02.2022
(51) Int. Cl.: A61N 1/36

(54) **NEUROSTIMULATION TITRATION AND PROGRAMMING BASED ON PATIENT FEEDBACK**
NEUROSTIMULATIONSTITRATION UND -PROGRAMMIERUNG AUF BASIS VON PATIENTENFEEDBACK
TITRAGE ET PROGRAMMATION DE NEUROSTIMULATION BASÉS SUR UNE RÉTROACTION DE PATIENT

(30) Priority: 24.02.2021 US 202163152863 P
(43) Date of publication of application: 03.01.2024
(73) Proprietor: Medtronic, Inc., Minneapolis, Minnesota 55432 (US)
(72) Inventor: SHARMA, Vinod, Minneapolis, Minnesota 55432 (US); CLELAND, Andrew J., Minneapolis, Minnesota 55432 (US); TORGERSON, Nathan A., Minneapolis, Minnesota 55432 (US); ISAACSON, Benjamin P., Minneapolis, Minnesota 55432 (US); KRAMER, Jeffery M., Minneapolis, Minnesota 55432 (US); HINCAPIE, Juan G., Minneapolis, Minnesota 55432 (US); KELLEY, Brooke G., Minneapolis, Minnesota 55432 (US); LITVAK, Leonid M., Minneapolis, Minnesota 55432 (US); DASSBACH GREEN, Paula Andrea Elma, Minneapolis, Minnesota 55432 (US); CASE, Michelle A., Minneapolis, Minnesota 55432 (US)
(74) Representative: Zimmermann & Partner Patentanwälte mbB
(86) International application number: PCT/US2022/070815
(87) International publication number: WO 2022/183198

(56) References cited:
- WO-A1-2018/080753
- US-A1- 2016 175 594
- US-A1- 2018 078 769
- US-A1- 2018 154 144
- US-A1- 2019 388 692

## Description

### TECHNICAL FIELD

This disclosure generally relates to medical devices, and more specifically, electrical stimulation.

### BACKGROUND

Electrical stimulation devices, sometimes referred to as neurostimulators or neurostimulation devices, may be external to or implanted within a patient, and configured to deliver electrical stimulation therapy to various tissue sites to treat a variety of symptoms or conditions such as chronic pain, tremor, Parkinson's disease, epilepsy, or other neurological disorders, urinary or fecal incontinence, sexual dysfunction, obesity, or gastroparesis. An electrical stimulation device may deliver electrical stimulation therapy via electrodes, e.g., carried by one or more leads, positioned proximate to target locations associated with the brain, the spinal cord, pelvic nerves, tibial nerves, peripheral nerves, the gastrointestinal tract, or elsewhere within a patient. Stimulation proximate the spinal cord, proximate the sacral nerve, within the brain, and proximate peripheral nerves is often referred to as spinal cord stimulation (SCS), sacral neuromodulation (SNM), deep brain stimulation (DBS), and peripheral nerve stimulation (PNS), respectively.

A physician or clinician may select values for a number of programmable stimulation parameters in order to define the electrical stimulation therapy to be delivered by the implantable stimulator to a patient. For example, the physician or clinician may select one or more electrodes, polarities of selected electrodes, a voltage or current amplitude, a pulse width, and a pulse frequency as stimulation parameters. A set of therapy stimulation parameters, such as a set including electrode combination, electrode polarity, amplitude, pulse width and pulse frequency, may be referred to as a therapy program in the sense that they define the electrical stimulation therapy to be delivered to the patient. Documents WO 2018/080753 A1 and US 2018/154144 A1 relate to electrical stimulation devices.

### SUMMARY

The invention is defined by the independent claims. In general, the disclosure describes techniques for titrating neurostimulation from a first amount over a period of time to a second, lesser, amount over the period of time based on patient feedback. In some examples, the amount of neurostimulation over a period of time is controlled by both the stimulation dose and the cycling of the stimulation dosing, e.g., the frequency and amount of time the stimulation dosing is "on" or being delivered relative to the amount of time the stimulation dosing is "off" or not being delivered. In some examples, the amount of stimulation may be reduced over time by reducing one or both of the frequency and amount of time the dosing is on based on patient feedback.

A neurostimulation device, external programmer, or remote programming device may receive patient feedback from one or more sensing and/or patient-input devices, either directly or via network connections, and perform, direct or control, based on the patient feedback, automatic control of one or more neurostimulation stimulation cycling parameters. In this manner, a neurostimulation device, external programmer, or remote programming device may select, adjust or control one or more neurostimulation stimulation cycling parameters based on the patient feedback to eliminate, reduce, alleviate, or delay stimulation tolerance and to increase the battery life of the neurostimulation device.

In one example, this disclosure describes a method of titrating a therapy that includes determining, for a patient, a first cycling of electric stimulation doses; delivering, via an implanted device, the electric stimulation doses to the patient according the determined first cycling; receiving patient feedback representing a response of the patient to the electric stimulation doses delivered according to the first cycling; determining, based on the patient feedback, a second cycling of the electric stimulation doses; and delivering, via the implanted device, the electric stimulation doses to the patient according the determined second cycling, wherein delivering the electric stimulation doses for the patient according to the determined second cycling consumes less power of the implanted device than delivering the electric stimulation doses for the patient according to the determined first cycling.

In another example, this disclosure describes a system that includes electrical stimulation circuitry configured to generate electrical stimulation; electrodes configured to deliver the electrical stimulation to a patient; and processing circuitry configured to: determine, for a patient, a first cycling of electric stimulation doses; deliver the electric stimulation doses according the determined first cycling; receive patient feedback representing a response of the patient to the electric stimulation doses delivered according to the first cycling; determine, based on the patient feedback, a second cycling of the electric stimulation doses; and deliver the electric stimulation doses to the patient according the determined second cycling, wherein delivering the electric stimulation doses for the patient according to the determined second cycling consumes less power of the implanted device than delivering the electric stimulation doses for the patient according to the determined first cycling.

In another example, this disclosure describes a computer readable medium comprising instructions that when executed cause one or more processors to: determine, for a patient, a first cycling of electric stimulation doses; deliver the electric stimulation doses according the determined first cycling; receive patient feedback representing a response of the patient to the electric stimulation doses delivered according to the first cycling; determine, based on the patient feedback, a second cycling of the electric stimulation doses; and deliver the electric stimulation doses to the patient according the determined second cycling, wherein delivering the electric stimulation doses for the patient according to the determined second cycling consumes less power of the implanted device than delivering the electric stimulation doses for the patient according to the determined first cycling, wherein one or both of an amount of on-time of each cycle or a frequency of the second cycling is less than one or both of a corresponding amount of on-time of each cycle or a corresponding frequency of the first cycling, wherein the patient feedback comprises one or more of a physiological signal, a patient input, patient posture data, an evoked compound action potential (ECAP), a local field potential (LFP), a heart rate, a heart rate variability, a blood flow, a galvanic skin response, a network excitability, a pain response, a pain score, an area of pain, an amount of paresthesia, an area of paresthesia, or a voiding rate.

The summary is intended to provide an overview of the subject matter described in this disclosure. It is not intended to provide an exclusive or exhaustive explanation of the systems, device, and methods described in detail within the accompanying drawings and description below. Further details of one or more examples of this disclosure are set forth in the accompanying drawings and in the description below. Other features, objects, and advantages will be apparent from the description and drawings, and from the claims.

### BRIEF DESCRIPTION OF DRAWINGS

FIG. 1 is a conceptual diagram illustrating an example system that includes an implantable medical device (IMD) in the form of a neurostimulation device configured to deliver spinal cord stirmilation (SCS), an external programmer, and one or more sensing devices in accordance with one or more techniques of this disclosure.
FIG. 2A is a block diagram illustrating an example of an IMD in the form of a neurostimulation device, in accordance with one or more techniques of this disclosure.
FIG. 2B is a block diagram illustrating an example of an IMD in the form of a neurostimulation device, in accordance with one or more techniques of this disclosure.
FIG. 3 is a block diagram illustrating an example of an external programmer suitable for use with the IMD of FIG. 2, in accordance with one or more techniques of this disclosure.
FIG. 4 is a flow diagram illustrating an example method of titrating a therapy, in accordance with one or more techniques of this disclosure.
FIG. 5 is a plot of an example of titrating electric stimulation doses, in accordance with one or more techniques of this disclosure.
FIG. 6 is a plot of another example of titrating electric stimulation doses, in accordance with one or more techniques of this disclosure.
FIG. 7 is a plot of another example of titrating electric stimulation doses, in accordance with one or more techniques of this disclosure.
FIG. 8 is a plot of another example of titrating electric stimulation doses, in accordance with one or more techniques of this disclosure.
FIG. 9 is a plot of another example of titrating electric stimulation doses, in accordance with one or more techniques of this disclosure.

### DETAILED DESCRIPTION

Electrical stimulation therapy (e.g., including spinal cord stimulation, tibial nerve stimulation, etc.) may provide pain relief and/or other therapeutic benefits. In some circumstances, constant delivery of electrical stimulation doses may be required to achieve the desired pain relief and/or other therapeutic benefits. In other circumstances, electrical stimulation may have a durable effect such that constant delivery of electrical stimulation is not required to achieve the desired pain relief and/or other therapeutic benefits. Where electrical stimulation has such a durable effect, a device may deliver electrical stimulation to a patient in accordance with a treatment program that proscribes on periods in which the device delivers electrical stimulation doses of the treatment program and off periods in which the device does not deliver electrical stimulation doses of the treatment program.

In accordance with one or more techniques of this disclosure, an electrical stimulation system may titrate the cycling of the stimulation therapy based on patient feedback. For instance, the electrical stimulation system may titrate the cycling such that the stimulation dosing is cycled on (e.g., delivered) when needed and may otherwise be cycled off. For example, if a patient is doing well, the system may titrate the cycling such that electrical stimulation doses are delivered less often (e.g., therapy may be cycled to be in the off state more often and/or for a longer duration). By increasing the amount of time the therapy is in the off state, the electrical stimulation system may conserve battery life and/or reduce the development of a tolerance to the stimulation therapy. If a patient started to do worse, the system may titrate the cycling such that the stimulation doses are delivered more often (e.g., therapy may be cycled to be in the on state more often and/or for a longer duration).

The system may receive patient feedback including subjective feedback and/or objective feedback. The system may receive subjective patient feedback including a binary pain response (e.g., press a button when there is pain), a pain score, an area of pain, an amount of paresthesia, or an area of paresthesia pain scores, voids per day, and the like. The system may receive objective patient feedback including biomarkers, physiological signals, or electronically sensed outcomes such as an evoked compound action potential (ECAP), a local field potential (LFP), a heart rate, a heart rate variability, blood flow, or a galvanic skin response, or a patient posture or behavior, such as a patient position, a patient movement, a patient movement history over a predetermined amount of time, a history of patent-selected stimulation parameters over a predetermined amount of time.

In some examples, the system may alter settings or the occurrence of the applied therapy so that the therapy is not provided more than necessary, e.g., to save battery life, but also to provide the therapy frequently enough so that the outcome is consistent and beneficial when the therapy is not being provided.

In some examples, e.g., for fully implantable systems, a patient may use a user interface on a patient's phone or on their patient programmer to input feedback, such as pain scores. The system may titrate the cycling by reducing the cycle frequency of the electric stimulation doses and/or changing the duty cycle of the cycling to shorten the on-time (or lengthen the off-time) to balance the therapy between therapeutic effects, potential development of tolerance, and device battery life based on the feedback and/or feedback trends. In some examples, cycling and/or tapering titration of cycling may be used with durable-effect therapies with long (days) or short (minutes) durable effect timescales.

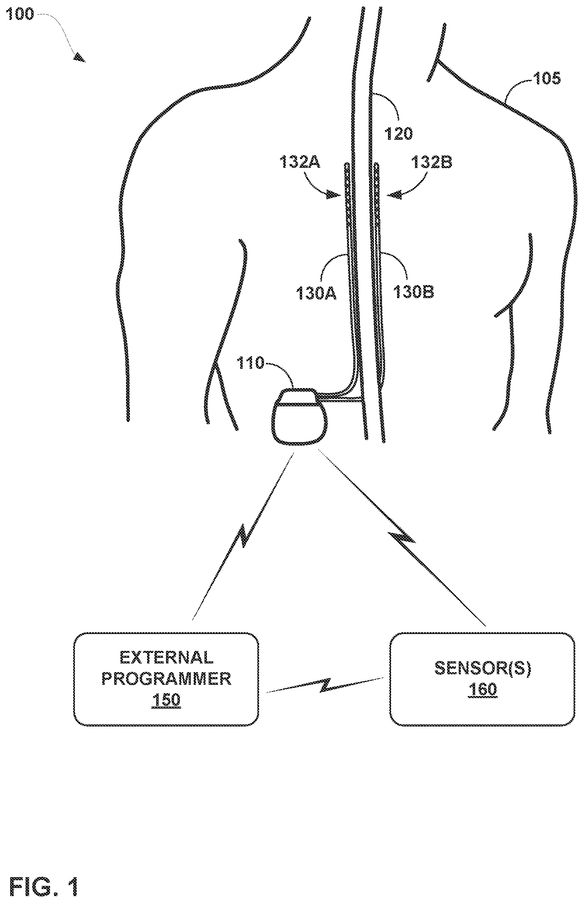

Systems and methods for titrating a neurostimulation therapy based on patient feedback are described herein. The system may include a stimulator system that interacts with a stimulator programmer. FIG. 1 is a conceptual diagram illustrating an example system 100 that includes an implantable medical device (IMD) 110 configured to deliver spinal cord stimulation (SCS) therapy, processing circuitry 140, an external programmer 150, and one or more sensors 160, in accordance with one or more examples of this disclosure. Processing circuitry 140 may include one or more processors configured to perform various operations of IMD 110. Although the examples described in this disclosure are generally applicable to a variety of medical devices including external devices and IMDs, application of such techniques to IMDs and, more particularly, implantable electrical stimulators (e.g., neurostimulators) will be described for purposes of illustration. More particularly, the disclosure will refer to an implantable SCS system for purposes of illustration, but without limitation as to other types of neurostimulation devices or other therapeutic applications of neurostimulation, including an external neurostimulator. For example, the system may not be a fully implanted system where the pulse generator is external to the patient and stimulation is transmitted transdermally. In one or more examples, the stimulators may be configured to deliver peripheral nerve stimulation or spinal nerve root stimulation.

As shown in FIG. 1, system 100 includes an IMD 110, leads 130A and 130B, and external programmer 150 shown in conjunction with a patient 105, who is ordinarily a human patient. In the example of FIG. 1, IMD 110 is an implantable electrical stimulator that is configured to generate and deliver electrical stimulation therapy to patient 105, e.g., for relief of chronic pain or other symptoms, via one or more electrodes 132A, 1 32B of leads 130A and/or 130B, respectively. In the example of FIG. 1, each lead 130A, 130B includes eight electrodes 132A, 132B respectively, although the leads may each have a different number of electrodes. Leads 130A, 130B may be referred to collectively as "leads 130" and electrodes 132A, 132B may be referred to collectively as "electrodes 132." In other examples, IMD 110 may be coupled to a single lead carrying multiple electrodes or more than two leads each carrying multiple electrodes.

IMD 110 may be a chronic electrical stimulator that remains implanted within patient 105 for weeks, months, or years. In other examples, IMD 110 may be a temporary, or trial, stimulator used to screen or evaluate the efficacy of electrical stimulation for chronic therapy. In one example, IMD 110 is implanted within patient 105, while in another example, IMD 110 is an external device coupled to one or more leads percutaneously implanted within the patient. In some examples, IMD 110 uses electrodes on one or more leads, while in other examples, IMD 110 may use one or more electrodes on a lead or leads and one of more electrodes on a housing of the IMD. In further examples, IMD 110 may be leadless and instead use only electrodes carried on a housing of the IMD.

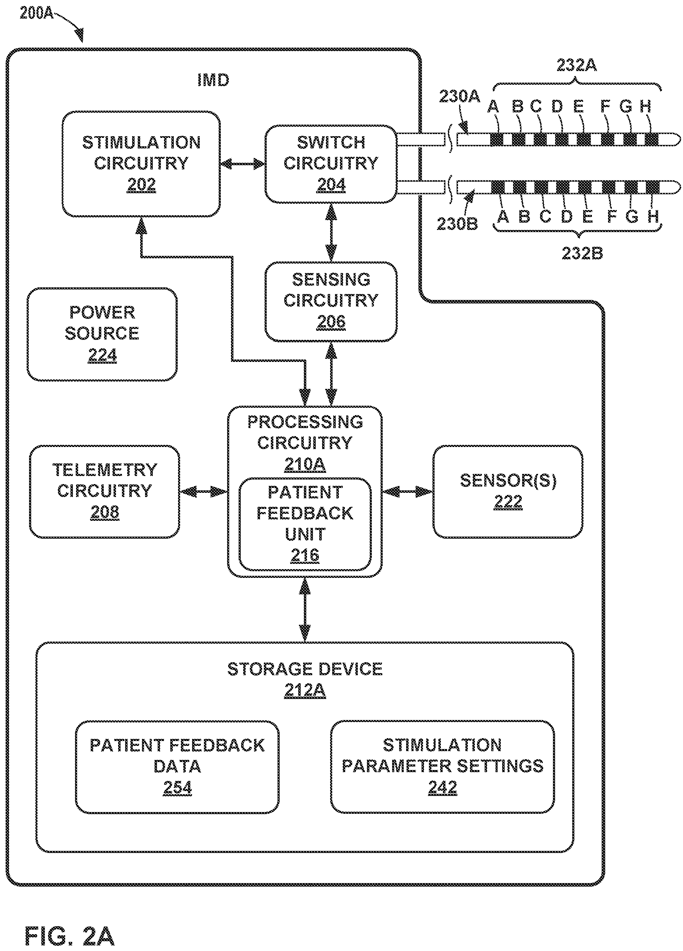

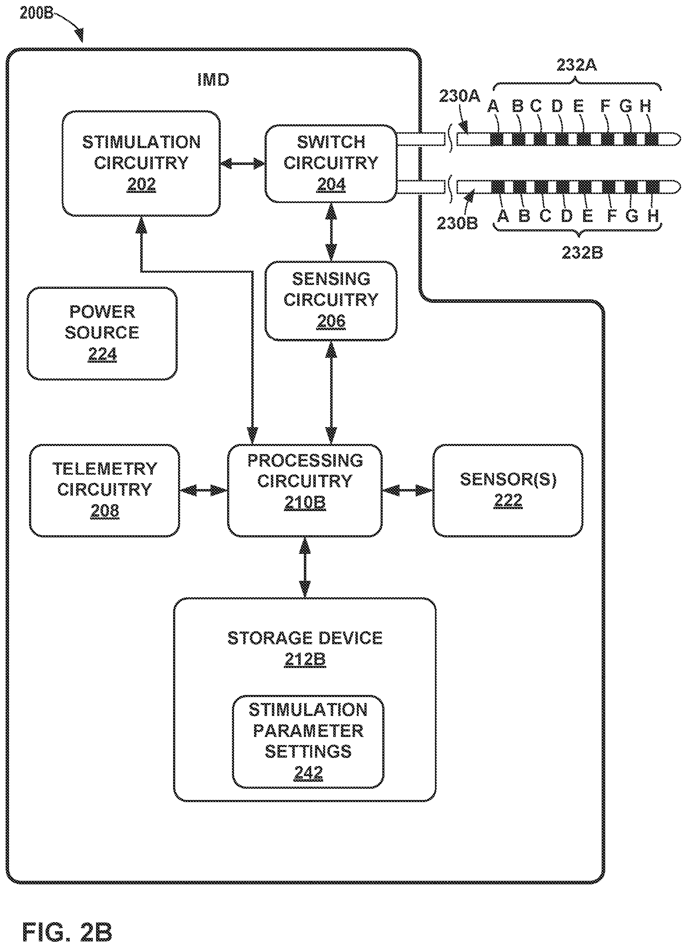

IMD 110 may be constructed of any polymer, metal, or composite material sufficient to house the components of IMD 110 (e.g., components illustrated in FIG. 2A, 2B) within patient 105. In this example, IMD 110 may be constructed with a biocompatible housing, such as titanium or stainless steel, or a polymeric material such as silicone, polyurethane, or a liquid crystal polymer, and surgically implanted at a site in patient 105 near the pelvis, abdomen, or buttocks. In other examples, IMD 110 may be implanted at other suitable sites within patient 105, which may depend, for example, on the target site within patient 105 for the delivery of electrical stimulation therapy. The outer housing of IMD 110 may be configured to provide a hermetic seal for components, such as a rechargeable or non-rechargeable power source. In addition, in some examples, the outer housing of IMD 110 is selected from a material that facilitates receiving energy to charge the rechargeable power source.

In the example of FIG. 1, electrical stimulation energy, which may be delivered as regulated current or regulated voltage-based pulses, is delivered from IMD 110 to one or more target tissue sites of patient 105 via leads 130 and electrodes 132. Leads 130 position electrodes 132 adjacent to target tissue of spinal cord 120. One or more of the electrodes 132 may be disposed at a distal tip of a lead 130 and/or at other positions at intermediate points along the lead. Leads 130 may be implanted and coupled to IMD 110. The electrodes 132 may transfer electrical stimulation generated by an electrical stimulation generator in IMD 110 to tissue of patient 105. Although leads 130 may each be a single lead, a lead 130 may include a lead extension or other segments that may aid in implantation or positioning of lead 130.

The electrodes 132 of leads 130 may be electrode pads on a paddle lead, circular (e.g., ring) electrodes surrounding the body of the lead, conformable electrodes, cuff electrodes, segmented electrodes (e.g., electrodes disposed at different circumferential positions around the lead instead of a continuous ring electrode), any combination thereof (e.g., ring electrodes and segmented electrodes) or any other type of electrodes capable of forming unipolar, bipolar or multipolar electrode combinations for therapy. Ring electrodes arranged at different axial positions at the distal ends of lead 130 will be described for purposes of illustration. Deployment of electrodes via leads 130 is described for purposes of illustration, but electrodes may be arranged on a housing of IMD 110, e.g., in rows and/or columns (or other arrays or patterns), as surface electrodes, ring electrodes, or protrusions.

Neurostimulation stimulation parameters defining the electrical stimulation pulses delivered by IMD 110 through electrodes 132 of leads 130 may include information identifying which electrodes have been selected for delivery of the stimulation pulses according to a stimulation program and the polarities of the selected electrodes (the electrode combination), and voltage or current amplitude, pulse rate (i.e., frequency), and pulse width of the stimulation pulses. The neurostimulation stimulation parameters may further include a cycling parameter that specifies when, or how long, stimulation is turned on and off. Neurostimulation stimulation parameters may be programmed prior to delivery of the neurostimulation pulses, manually adjusted based on user input, or automatically controlled during delivery of the neurostimulation pulses, e.g., based on sensed conditions.

Although the example of FIG. 1 is directed to SCS therapy, e.g., to treat pain, in other examples, system 100 may be configured to treat other conditions that may benefit from neurostimulation therapy. For example, system 100 may be used to treat tremor, Parkinson's disease, epilepsy, or other neurological disorders, urinary or fecal incontinence, sexual dysfunction, obesity, or gastroparesis, or psychiatric disorders such as depression, mania, obsessive compulsive disorder, or anxiety disorders. Hence, in some examples, system 100 may be configured to deliver sacral neuromodulation (SNM), deep brain stimulation (DBS), peripheral nerve stimulation (PNS), or other stimulation, such as peripheral nerve field stimulation (PNFS), cortical stimulation (CS), gastrointestinal stimulation, or any other stimulation therapy capable of treating a condition of patient 105. In some examples, system 100 may be configured where the electrical stimulation includes stimulation parameters to deliver therapy to address a condition of one or more of painful diabetic neuropathy (PDN), peripheral vascular disease (PVD), peripheral artery disease (PAD), complex regional pain syndrome (CRPS), angina pectoris (AP), leg pain, back pain or pelvic pain.

Leads 130 may include, in some examples, one or more sensors configured to sense one or more physiological stimulation parameters of patient 105, such as patient activity, pressure, temperature, posture, heart rate, blood flow or other characteristics. At least some of electrodes 132 may be used to sense electrical signals within patient 105, additionally or alternatively to delivering stimulation. IMD 110 is configured to deliver electrical stimulation therapy to patient 105 via selected combinations of electrodes carried by one or both of leads 130, alone or in combination with an electrode carried by or defined by an outer housing of IMD 110. The target tissue for the electrical stimulation therapy may be any tissue affected by electrical stimulation. In some examples, the target tissue includes nerves, smooth muscle or skeletal muscle. In the example illustrated by FIG. 1, the target tissue is tissue proximate spinal cord 120, such as within an intrathecal space or epidural space of spinal cord 120, or, in some examples, adjacent nerves that branch off spinal cord 120. Leads 130 may be introduced into spinal cord 120 in via any suitable region, such as the thoracic, cervical or lumbar regions.

Stimulation of spinal cord 120 may, for example, prevent pain signals from being generated and/or traveling through spinal cord 120 and to the brain of patient 105. Patient 105 may perceive the interruption of pain signals as a reduction in pain and, therefore, efficacious therapy results. In some examples, stimulation of spinal cord 120 may produce paresthesia which may reduce the perception of pain by patient 105, and thus, provide efficacious therapy results. In other examples, stimulation of spinal cord 120 may be effective in reducing pain with or without presenting paresthesia. In some examples, some electrical stimulation pulses may be directed to glial cells while other electrical stimulation (e.g., delivered by a different electrode combination and/or with different stimulation parameters) is directed to neurons. In other examples, stimulation of spinal cord 120 may be effective in promoting blood flow in one or more remote tissue locations, e.g., in a limb or appendage, thereby alleviating or reducing pain or other symptoms, or preventing or delaying onset of tissue damage or degeneration.

IMD 110 generates and delivers electrical stimulation therapy to a target stimulation site within patient 105 via the electrodes of leads 130 to patient 105 according to one or more therapy stimulation programs. A therapy stimulation program specifies values for one or more stimulation parameters that define an aspect of the therapy delivered by IMD 110 according to that program. For example, a stimulation therapy program that controls delivery of stimulation by IMD 110 in the form of stimulation pulses may define values for voltage or current pulse amplitude, pulse width, and pulse rate (e.g., pulse frequency) for stimulation pulses delivered by IMD 110 according to that program, as well as the particular electrodes and electrode polarities forming an electrode combination used to deliver the stimulation pulses. Hence, a stimulation therapy program may specify the location(s) at which stimulation is delivered and amplitude, pulse width and pulse rate of the stimulation. In some examples, a stimulation therapy program may specify cycling of the stimulation, e.g., in terms of that when, or how long, stimulation is turned on and off

A user, such as a clinician or patient 105, may interact with a user interface of an external programmer 150 to program IMD 110. Programming of IMD 110 may refer generally to the generation and transfer of commands, programs, or other information to control the operation of IMD 110. In this manner, IMD 110 may receive the transferred commands and programs from external programmer 150 to control electrical stimulation therapy. For example, external programmer 150 may transmit therapy stimulation programs, stimulation parameter adjustments, therapy stimulation program selections, user input, or other information to control the operation of IMD 110, e.g., by wireless telemetry or wired connection.

In some cases, external programmer 150 may be characterized as a physician or clinician programmer if it is primarily intended for use by a physician or clinician. In other cases, external programmer 150 may be characterized as a patient programmer if it is primarily intended for use by a patient. A patient programmer may be generally accessible to patient 105 and, in many cases, may be a portable device that may accompany patient 105 throughout the patient's daily routine, e.g., as a handheld computer similar to a tablet or smartphone. For example, a patient programmer may receive input from patient 105 when the patient wishes to terminate or change stimulation therapy. In general, a physician or clinician programmer may support selection and generation of programs by a clinician for use by IMD 110, and may take the form, for example, of a handheld computer (e.g., a tablet computer), laptop computer or desktop computer, whereas a patient programmer may support adjustment and selection of such programs by a patient during ordinary use. In other examples, external programmer 150 may include, or be part of, an external charging device that recharges a power source of IMD 110. In this manner, a user may program and charge IMD 110 using one device, or multiple devices.

IMD 110 and external programmer 150 may exchange information and may communicate via wireless communication using any techniques known in the art. Examples of communication techniques may include, for example, radiofrequency (RF) telemetry and inductive coupling, but other techniques are also contemplated. In some examples, external programmer 150 includes a communication head that may be placed proximate to the patient's body near the IMD 110 implant site to improve the quality or security of communication between IMD 110 and external programmer 130. Communication between external programmer 150 and IMD 110 may occur during power transmission or separate from power transmission.

IMD 110, in response to commands from external programmer 150, may deliver electrical stimulation therapy according to a plurality of therapy stimulation programs to a target tissue site of the spinal cord 120 of patient 105 via electrodes 132 on leads 130. In some examples, IMD 110 automatically modifies therapy stimulation programs as therapy needs of patient 1 05 evolve over time. For example, the modification of the therapy stimulation programs may cause the adjustment of at least one parameter of the plurality of stimulation pulses based on received information.

IMD 110 and/or external programmer 150 may receive information from one or more sensors 160, e.g., directly via wireless communication or indirectly from an intermediate server via a network connection. Sensor 160 may be positioned to sense one or more physiological responses at a selected location on patient 105. In some examples, sensor 160 may be positioned at, attached to or near tissue for a target anatomical area, e.g., at a limb or appendage, such as at or on a leg, toe, foot, arm, finger or hand of patient 105, e.g., to sense a galvanic skin response adjacent to placement of sensor 160. In some examples, sensor 160 may be attached to an appendage of the patient 105 to sense a physiological response associated with the appendage, e.g., by a clip-on mechanism, strap, elastic band and/or adhesive. In some examples, sensor 160 (or one of a plurality of sensors 160) may be implantable within patient 105, e.g., within a limb or appendage of the patient, near the spinal cord of the patient, within the brain of the patient, and the like.

In some examples, sensor 160 may be a physiological and/or patient posture or behavior sensor. For example, sensor 160 may be a heart rate monitor configured to detect and/or determine a heart rate and/or a heart rate variability. Sensor 160 may be configured to detect and/or determine a galvanic skin response, or to detect and/or determine a biopotential. Sensor 160 may be a thermometer configured to detect and/or determine a temperature of at least a part of the patient's anatomy. Sensor 160 may be configured to measure a pressure, e.g., a patient blood pressure, or to measure an impedance of at least a portion of the patient's anatomy. Sensor 160 may be a blood flow sensor that measures blood flow and provides information related to blood flow associated with tissue of the patient. For example, sensor 160 may provide blood flow values, or other information indicative of blood flow values or changes in blood flow values. The blood flow value may be an instantaneous blood flow measurement or may be a measurement of blood flow over a period of time such as average blood flow value, maximum blood flow value, minimum blood flow value during the period of time. In some examples, sensor 160 may be a microphone configured to detect/determine sounds of at least a portion of the patient's anatomy. In some examples, sensor 160 may at least partially comprise electrodes 132A, 132B. For example, sensor 160 may be configured to detect and/or determine ECAPs, LFPs, a network excitability, and the like. In some examples, sensor 160 may comprise and accelerometer configured to detect and/or determine a position and/or patient movement, a patient movement history over a predetermined amount of time, and the like. In some examples, sensor 160 may be a patient-input device, e.g., external programmer 150, a smartphone or computing device, or any other suitable device, configured to receive and communicate subjective patient feedback. For example, sensor 160 may be configured to receive a pain response, a pain score, an area of pain, an amount of paresthesia, an area of paresthesia, information relating to voiding and/or a voiding rate (e.g., voids per day), and the like. In some examples, sensor 160 may be an environmental sensor, such as a microphone, thermometer, hygrometer, pressure sensor, and the like, configured to detect and/or determine sounds, temperatures, humidity and pressure, etc., of the environment in which the patient is located.

In accordance with one or more aspects of this disclosure, IMD 110 and/or external programmer 150 may be configured to titrate and/or taper a titration of electric stimulation therapy. For example, a physician or clinician, IMD 110, or external programmer 150 may be configured to determine a first cycling of electric stimulation doses. A "dose" of electric stimulation may comprise delivery of electric stimulation pulses according to one or more specified electric stimulation parameters, e.g., an electrode combination, an amplitude, a pulse frequency, and a pulse width. Cycling of the electric stimulation doses may comprise delivering a plurality of electric stimulation doses periodically or according to a schedule. In some examples, an electric stimulation cycle may comprise a cycle frequency, e.g., the rate at which electric stimulation doses are delivered, and/or a cycling duty cycle, e.g., the ratio of stimulation pulse delivery on-time to off-time during each electric stimulation cycle or period. The cycle frequency may be specified in a variety of manners, such as time between dose delivery (e.g., in seconds, minutes, hours, days, weeks, months, etc.), an amount of time for both on-time and off-time (e.g., on-time/off-time pairs in second, minutes, hours, days, weeks, months, etc.), as a schedule specifying both an on-time and an off-time for each does which may be the same or which may vary for each does, a continuous taper in which the off-time is increased at a constant or variable rate and/or an on-time is decreased at a constant or a variable rate, and the like.

IMD 110 may be configured to deliver one or more electric stimulation doses to the patient according to the determined first cycling, and IMD 110 and/or external programmer 150 may be configured to receive patient feedback representing a response of the patient to the one or more electric stimulation doses delivered according to the first cycling, e.g., via sensor 160. IMD 110 and/or external programmer 150 may be configured to determine a second cycling of the electric stimulation doses based on the received feedback, and IMD 110 may be configured to deliver one or more electric stimulation doses to the patient according the determined second cycling. In this way, IMD 110 may be configured to consume and/or use less electrical power when delivering the one or more electric stimulation doses for the patient according to the determined second cycling than delivering the one or more electric stimulation doses for the patient according to the determined first cycling, and to have an increased battery life. Additionally or alternatively, IMD 110 may be configured to reduce eliminate, reduce, alleviate, or delay stimulation tolerance when delivering the one or more electric stimulation doses for the patient according to the determined second cycling than delivering the one or more electric stimulation doses for the patient according to the determined first cycling.

FIG. 2A and 2B are block diagrams illustrating example configurations of components of an IMD 200A and an IMD 200B, respectively, in accordance with one or more techniques of this disclosure. IMD 200A and/or IMD 200B may be an example of IMD 110 of FIG. 1. In the examples shown in FIGS. 2A and 2B, IMD 200A and IMD 200B each include stimulation generation circuitry 202, switch circuitry 204, sensing circuitry 206, telemetry circuitry 208, sensor(s) 222, power source 224, lead 230A carrying electrodes 232A, which may correspond to lead 130A and electrodes 132A of FIG. 1, and lead 230B carrying electrodes 232B, which may correspond to lead 130B and electrodes 132B of FIG. 1. In the examples shown in FIG. 2A, IMD 200A includes processing circuitry 210A and storage device 212A, and in the example shown in FIG. 2B, IMD 200B includes processing circuitry 210B and storage device 212B. Processing circuitry 210A and/or 210B may include one or more processors configured to perform various operations of IMD 200A and/or IMD 200B.

In the examples shown in FIGS. 2A and 2B, storage devices 212A and 212B store stimulation parameter settings 242. In addition, as shown in FIG. 2A, storage device 212A may store patient feedback data 254 obtained directly or indirectly from one or more sensors 160 (FIG. 1) or from patient via a patient-input device. In this case, IMD 200A of FIG. 2A may process patient feedback information and select or adjust stimulation parameter settings, including cycling, based on the patient feedback information.

In one or more examples, such as shown in FIG. 2B, the IMD 200B may not store or receive the patient feedback information. Instead, external programmer 150 or another device may directly or indirectly select or adjust stimulation parameter settings based on patient feedback information and communicate the selected settings or adjustments to IMD 200B of FIG. 2B. In some examples, stimulation parameter settings 242 may include stimulation parameters (sometimes referred to as "sets of therapy stimulation parameters") for respective different stimulation programs selectable by the clinician or patient for therapy. In some examples, stimulation parameter settings 242 may include one or more recommended parameter settings. In this manner, each stored therapy stimulation program, or set of stimulation parameters, of stimulation parameter settings 242 defines values for a set of electrical stimulation parameters (e.g., a stimulation parameter set), such as electrode combination (selected electrodes and polarities), stimulation current or voltage amplitude, stimulation pulse width, pulse rate, or duty cycle. In some examples, stimulation parameter settings 242 may further include cycling information indicating when or how long stimulation is turned on and off, e.g., periodically and/or according to a schedule. For example, recommended parameter settings may indicate the stimulation to turn on for a certain period of time, and/or to turn off stimulation for a certain period of time. In another example, recommended cycle parameter settings may indicate for stimulation to turn on for a period of time without creating desensitization of the stimulation. In one or more examples, the recommended parameter settings may indicate stimulation to occur at a certain time of day, for example when the patient is typically awake or active, or sleeping. In one or more examples, recommended parameter settings relate to when the patient has a certain posture, for example only deliver stimulation when the patient is in a supine position.

Stimulation generation circuitry 202 includes electrical stimulation circuitry configured to generate electrical stimulation and generates electrical stimulation pulses selected to alleviate symptoms of one or more diseases, disorders or syndromes. While stimulation pulses are described, stimulation signals may take other forms, such as continuous-time signals (e.g., sine waves) or the like. The electrical stimulation circuitry may reside in an implantable housing, for example of the IMD. Each of leads 230A, 230B may include any number of electrodes 232A, 232B. The electrodes are configured to deliver the electrical stimulation to the patient. In the example of FIGS. 2A and 2B, each set of electrodes 232A, 232B includes eight electrodes A-H. In some examples, the electrodes are arranged in bipolar combinations. A bipolar electrode combination may use electrodes carried by the same lead 230A, 230B or different leads. For example, an electrode A of electrodes 232A may be a cathode and an electrode B of electrodes 232A may be an anode, forming a bipolar combination. Switch circuitry 204 may include one or more switch arrays, one or more multiplexers, one or more switches (e.g., a switch matrix or other collection of switches), or other electrical circuitry configured to direct stimulation signals from stimulation generation circuitry 202 to one or more of electrodes 232A, 232B, or directed sensed signals from one or more of electrodes 232A, 232B to sensing circuitry 206. In some examples, each of the electrodes 232A, 232B may be associated with respective regulated current source and sink circuitry to selectively and independently configure the electrode to be a regulated cathode or anode. Stimulation generation circuitry 202 and/or sensing circuitry 206 also may include sensing circuitry to direct electrical signals sensed at one or more of electrodes 232A, 232B.

Sensing circuitry 206 may be configured to monitor signals from any combination of electrodes 232A, 232B. In some examples, sensing circuitry 206 includes one or more amplifiers, filters, and analog-to-digital converters. Sensing circuitry 206 may be used to sense physiological signals, such as ECAP signals and/or LFP signals. In some examples, sensing circuitry 206 detects ECAP and/or LFP signals from a particular combination of electrodes 232A, 232B. In some cases, the particular combination of electrodes for sensing ECAP and/or LFP signals includes different electrodes than a set of electrodes 232A, 232B used to deliver stimulation pulses. Alternatively, in other cases, the particular combination of electrodes used for sensing ECAP and/or LFP signals includes at least one of the same electrodes as a set of electrodes used to deliver stimulation pulses to patient 105. Sensing circuitry 206 may provide signals to an analog-to-digital converter, for conversion into a digital signal for processing, analysis, storage, or output by processing circuitry 210.

Telemetry circuitry 208 supports wireless communication between IMD 200A and/or IMD 200B and an external programmer or another computing device under the control of processing circuitry 210. Processing circuitry 210A and/or 210B of IMD 200A and/or IMD 200B, respectively, may receive, as updates to programs, values for various stimulation parameters such as amplitude and electrode combination, from the external programmer via telemetry circuitry 208. Processing circuitry 210A and/or 210B of IMD 200 A and/or IMD 200B, respectively, may store updates to the stimulation parameter settings 242 or any other data in storage device 212. Telemetry circuitry 208 in IMD 200A and/or IMD 200B, as well as telemetry circuits in other devices and systems described herein, such as the external programmer and patient feedback sensing system, may accomplish communication by radiofrequency (RF) communication techniques. In addition, telemetry circuitry 208 may communicate with an external medical device programmer via proximal inductive interaction of IMD 200A and/or IMD 200B with the external programmer, where the external programmer may be one example of external programmer 150 of FIG. 1. Accordingly, telemetry circuitry 208 may send information to the external programmer on a continuous basis, at periodic intervals, or upon request from IMD 110 or the external programmer.

Processing circuitry 210A and/or 210B may include one or more processors, such as any one or more of a microprocessor, a controller, a digital signal processor (DSP), an application specific integrated circuit (ASIC), a field-programmable gate array (FPGA), discrete logic circuitry, or any other processing circuitry configured to provide the functions attributed to processing circuitry 210A and/or 210B herein may be embodied as firmware, hardware, software or any combination thereof. Processing circuitry 210A and/or 210B controls stimulation generation circuitry 202 to generate stimulation signals according to stimulation parameter settings 242. In some examples, processing circuitry 210A and/or 210B may execute other instructions stored in storage device 212A and/or 212B, respectively, to apply stimulation parameters specified by one or more of programs, such as amplitude, pulse width, pulse rate, and pulse shape of each of the stimulation signals.

In the illustrated example of FIG. 2A, processing circuitry 210A includes a patient feedback unit 216 to process the patient feedback information. Patient feedback unit 216 may represent an example of a portion of processing circuitry configured to process patient feedback information received from a patient feedback sensor, such as sensor 160, and/or a patient-input device, such as external programmer 150 or a patient device such as the patient's phone and/or computing device. In the example of FIG. 2B, the processing of patient feedback information occurs in a device other than IMD 200B. Referring again to FIG. 2A, the patient feedback unit 216, discussed further below, receives information regarding the patient feedback data, such as information relating to sensed and/or received patient feedback associated with the efficacy of the electrical stimulation therapy, and controls the electrical stimulation circuitry 202 to deliver the electrical stimulation to the patient based on the received information, where the indications of the received information may be stored in a storage device. Processing circuitry 210A and/or 210B also controls stimulation generation circuitry 202 to generate and apply the stimulation signals to selected combinations of electrodes 232A, 232B. In some examples, stimulation generation circuitry 202 includes a switch circuit (instead of, or in addition to, switch circuitry 204) that may couple stimulation signals to selected conductors within leads 230, which, in turn, deliver the stimulation signals across selected electrodes 232A, 232B. Such a switch circuit may selectively couple stimulation energy to selected electrodes 232A, 232B and to selectively sense bioelectrical neural signals of a spinal cord of the patient with selected electrodes 232A, 232B. In other examples, however, stimulation generation circuitry 202 does not include a switch circuit and switch circuitry 204 does not interface between stimulation generation circuitry 202 and electrodes 232A, 232B. In these examples, stimulation generation circuitry 202 may include a plurality of pairs of current sources and current sinks, each connected to a respective electrode of electrodes 232A, 232B. In other words, in these examples, each of electrodes 232A, 232B is independently controlled via its own stimulation circuit (e.g., via a combination of a regulated current source and sink), as opposed to switching stimulation signals between different electrodes of electrodes 232A, 232B.

Storage device 212A and/or 212B may be configured to store information within IMD 200A and/or 200B, respectively, during operation. Storage device 212A and/or 212B may include a computer-readable storage medium or computer-readable storage device. In some examples, storage device 212A and/or 212B includes one or more of a short-term memory or a long-term memory. Storage device 212A and/or 212B may include, for example, random access memories (RAM), dynamic random access memories (DRAM), static random access memories (SRAM), magnetic discs, optical discs, flash memories, or forms of electrically programmable memories (EPROM) or electrically erasable and programmable memories (EEPROM). In some examples, storage device 212A and/or 212B is used to store data indicative of instructions, e.g., for execution by processing circuitry 210A and/or 210B, respectively. As discussed above, storage device 212A and/or 212B is configured to store stimulation parameter settings 242.

Power source 224 is configured to deliver operating power to the components of IMD 200A and/or 200B. Power source 224 may include a battery and a power generation circuit to produce the operating power. In some examples, the battery is rechargeable to allow extended operation. In some examples, recharging is accomplished through proximal inductive interaction between an external charger and an inductive charging coil within IMD 200A and/or 200B. Power source 224 may include any one or more of a plurality of different battery types, such as nickel cadmium batteries and lithium ion batteries.

In some examples as shown in FIG. 2A, the processing circuitry 210A of the IMD 200A directs delivery of electrical stimulation by the electrodes 232A, 232B of leads 230A, 230B, receives information relating to patient feedback from the patient feedback sensors or patient-input device, and generates output based on the received information. The patient feedback unit 216 may use patient feedback information to develop recommended electrical stimulation parameters or adjustments which are outputted to a user, where the user can use the indications or one or more recommended stimulation parameters to program the IMD 200A, e.g., by selecting or accepting the recommendations as stimulation parameter settings to be used by IMD 200A. For example, a particular cycling and/or a set of stimulation parameters are recommended to a user and presented to the user via the programmer. The user may accept the recommended cycling and/or one or more recommended stimulation parameters, and the programmer programs IMD 200A to implement and deliver stimulation with the selected electrode combination and/or stimulation parameters.

Processing circuitry 210A and/or 210B controls stimulation circuitry 202 to deliver stimulation energy with stimulation parameters specified by one or more stimulation parameter settings 242 stored on storage device 212A and/or 212B and, in the example of FIG. 2A, to collect patient feedback information pertaining to the stored stimulation parameter settings 242. Processing circuitry 210A and/or 210B collects this patient feedback information by receiving the information via telemetry from a remote patient feedback sensor and/or patient-input device at a remote site. Processing circuitry 210A may also control stimulation circuitry 202 to test different parameter settings and record corresponding patient feedback data for each selected combination, and test different parameter settings as they compare to sensed and/or patent-input patient feedback. For example, processing circuitry 210A directs stimulation circuitry 202 to deliver stimulation via a particular cycling and the patient feedback unit 216 collects the corresponding patient feedback data from telemetry circuitry 208. The patient feedback data 254 for this test may be stored in the storage device 212A. Processing circuitry 210A may adjust the previously tested cycling of the stimulation delivered via the electrode combination to a different cycling and collect the corresponding patient feedback data from the patient feedback sensor and/or patient-input device in response to stimulation with the adjusted cycling. The patient feedback data received for the stimulation at the changed stimulation parameter, in this example, cycling, would be saved in the storage device 212A and may be output to a user. The processing circuitry 210A may continue to shift the cycling by either increasing or decreasing the cycling frequency and/or cycling duty cycle, and recording the respective patient feedback data, which is stored on the storage device 212A and the information may be output to a user. While the example of cycling is provided, processing circuitry 210A may direct stimulation circuitry 202 to step through various incremental settings of other stimulation parameters, such as stimulation amplitude, pulse width, stimulation frequency, and record the respective patient feedback information for each stepped value. In one or more examples, processing circuitry 210A may direct stimulation circuitry to turn on for a certain period of time, and/or to turn off for a period of time, or to turn on at a certain time of day and record the respective patient feedback. Stimulation circuitry 202 may shift more than one stimulation parameter for each test and collect sensed patient feedback information for each of the multiple shifted stimulation parameters.

In some examples, the patient feedback unit 216 processes the patient feedback information to perform closed-loop control of the stimulation parameters based on the patient feedback information. The patient feedback unit 216 may store the patient feedback data 254 in storage device 212A. For example, patient feedback unit 216 may select or adjust one or more settings of parameter values, such as electrode combination, amplitude, pulse width or pulse rate, or cycling in response to patient feedback information. The patient feedback information may be collected when electrical stimulation is not delivered or upon delivery of electrical stimulation.

In some examples, the processing circuitry 210A and/or 21B of the IMD 200A and/or 210B, respectively, directs delivery of electrical stimulation of the electrodes 232A, 232B, and receives information relating to patient feedback from one or more patient feedback sensors 160, either directly (e.g., in the case of processing circuitry 210A) or via external controller (e.g., in the case of processing circuitry 210B), and controls the delivery of electrical stimulation of the electrodes 232A, 232B based on the received information in a closed loop setting. The patient feedback information may be received via the telemetry circuitry 208 either directly or indirectly from the patient feedback sensor 160 (FIG. 1). In an example, the IMD 200A and/or IMD 200B may receive the patient feedback information from an intermediate device other than the patient feedback sensor, such as external programmer 150.

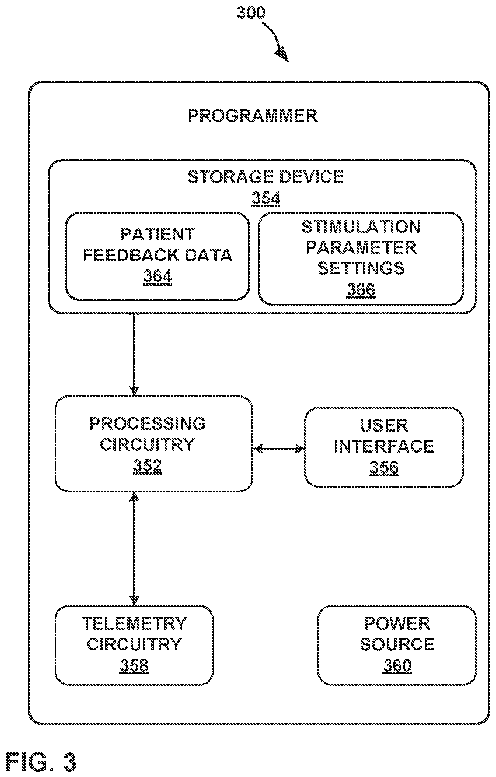

FIG. 3 is a block diagram illustrating an example configuration of components of an example external programmer 300. External programmer 300 may be an example of external programmer 150 of FIG. 1. Although external programmer 300 may generally be described as a hand-held device, such as a tablet computer or smartphone-like device, external programmer 300 may be a larger portable device, such as a laptop computer, or a more stationary device, such as a desktop computer. In addition, in other examples, external programmer 300 may be included as part of an external charging device or include the functionality of an external charging device, e.g., to recharge a battery or batteries associated with IMD 200. As illustrated in FIG. 3, external programmer 300 may include processing circuitry 352, storage device 354, user interface 356, telemetry circuitry 358, and power source 360. In some examples, storage device 354 may store instructions that, when executed by processing circuitry 352, cause processing circuitry 352 and external programmer 300 to provide the functionality ascribed to external programmer 300 throughout this disclosure. Each of these components, circuitry, or modules, may include electrical circuitry that is configured to perform some, or all of the functionality described herein. For example, processing circuitry 352 may include processing circuitry configured to perform the processes discussed with respect to processing circuitry 352.

In general, external programmer 300 includes any suitable arrangement of hardware, alone or in combination with software and/or firmware, to perform the techniques attributed to external programmer 300, and processing circuitry 352, user interface 356, and telemetry circuitry 358 of external programmer 300. In various examples, processing circuitry 352, telemetry circuitry 358, or other circuitry of external programmer 300 may include one or more processors, such as one or more microprocessors, DSPs, ASICs, FPGAs, or any other equivalent integrated or discrete logic circuitry, as well as any combinations of such components. External programmer 300 also, in various examples, may include a storage device 354, such as RAM, ROM, PROM, EPROM, EEPROM, flash memory, a hard disk, a CD-ROM, including executable instructions for causing the one or more processors to perform the actions attributed to them. Moreover, although processing circuitry 352 and telemetry circuitry 358 are described as separate modules, in some examples, processing circuitry 352 and telemetry circuitry 358 are functionally integrated. In some examples, processing circuitry 352, telemetry circuitry 358 or other circuitry of external programmer 300 may correspond to individual hardware units, such as ASICs, DSPs, FPGAs, or other hardware units.

The processing circuitry 352 is configured to direct delivery of electrical stimulation, receive information relating patient feedback. In some examples, the processing circuitry 352 is configured to control the electrical stimulation circuitry to deliver the electrical stimulation based on the patient feedback information in a closed loop basis by directing the IMD to use particular stimulation parameters.

Storage device 354 (e.g., a storage device) may, in some examples, store instructions that, when executed by processing circuitry 352, cause processing circuitry 352 and external programmer 300 to provide the functionality ascribed to external programmer 300 throughout this disclosure. For example, storage device 354 may include instructions that cause processing circuitry 352 to obtain a parameter set from memory or receive user input and send a corresponding command to IMD 200, or instructions for any other functionality. In addition, storage device 354 may include a plurality of programs, where each program includes a parameter set that defines therapy stimulation or control stimulation. Storage device 354 may also store data received from a medical device (e.g., IMD 110) and/or a remote sensing device. For example, storage device 354 may store data recorded at a sensing module of the medical device, and storage device 354 may also store data from one or more sensors of the medical device. In an example, storage device 354 may store data recorded at a remote sensing device such as patient feedback from one or more sensors and/or patient-input devices.

User interface 356 may include a button or keypad, lights, a speaker for voice commands, a display, such as a liquid crystal (LCD), light-emitting diode (LED), or organic light-emitting diode (OLED). In some examples, the display includes a touch screen. User interface 356 may be configured to display any information related to the delivery of electrical stimulation including output, for example, based on the patient feedback information. User interface 356 may also receive user input (e.g., indication of when the patient perceives stimulation, or a pain score perceived by the patient upon delivery of stimulation) via user interface 356. The user input may be, for example, in the form of pressing a button on a keypad or selecting an icon from a touch screen. The input may request starting or stopping electrical stimulation, the input may request a new electrode combination or a change to an existing electrode combination, or the input may request some other change to the delivery of electrical stimulation, such as a change in stimulation cycling, amplitude, pulse width or pulse rate.

Telemetry circuitry 358 may support wireless communication between the medical device and external programmer 300 under the control of processing circuitry 352. Telemetry circuitry 358 may also be configured to communicate with another computing device via wireless communication techniques, or direct communication through a wired connection. In some examples, telemetry circuitry 358 provides wireless communication via an RF or proximal inductive medium. In some examples, telemetry circuitry 358 includes an antenna, which may take on a variety of forms, such as an internal or external antenna.

Examples of local wireless communication techniques that may be employed to facilitate communication between external programmer 300 and IMD 110 include RF communication according to the 802.11 or Bluetooth ^{®} specification sets or other standard or proprietary telemetry protocols. In this manner, other external devices may be capable of communicating with external programmer 300 without needing to establish a secure wireless connection. As described herein, telemetry circuitry 358 may be configured to transmit a spatial electrode movement pattern or other stimulation parameters to IMD 110 for delivery of electrical stimulation therapy.

Power source 360 is configured to deliver operating power to the components of external programmer 300. Power source 360 may include a battery and a power generation circuit to produce the operating power. In some examples, the battery is rechargeable to allow extended operation. Recharging may be accomplished by electrically coupling power source 360 to a cradle or plug that is connected to an alternating current (AC) outlet. In addition, recharging may be accomplished through proximal inductive interaction between an extemal charger and an inductive charging coil within external programmer 300. In other examples, traditional batteries (e.g., nickel cadmium or lithium ion batteries) may be used. In addition, external programmer 300 may be directly coupled to an alternating current outlet to operate.

In some examples, the external programmer 300 or external control device directs delivery of electrical stimulation of an IMD, receives information relating to patient feedback, and generates output based on the received information, e.g., for evaluation of efficacy of stimulation parameters and/or to recommend or assist a user in programming stimulation parameters for delivery of electrical stimulation, or used as part of a closed loop control device to automatically adjust stimulation parameters using patient feedback information. In one or more examples, the control device generates output based on a first received information and a second received information via a user interface device.

Programmer 300 may be a patient programmer or a clinician programmer and receives patient feedback information such as patient feedback data 364. Programmer 300 receives patient feedback information and allows a user to interact with the processing circuitry 352 via user interface 356 in order to identify efficacious parameter settings, such as cycling and/or one or more other stimulation parameters using the patient feedback information. Programmer 300 further assists the user in programming a neurostimulation device by using the patient feedback information displayed on the user interface 356. In addition, programmer 300 may be used as part of a closed loop control device to automatically adjust stimulation parameters based at least on patient feedback information. In some examples, programmer 300 receives patient feedback information such as patient feedback data 364 from the patient feedback device and stores the patient feedback data in the storage device 354.

Programmer 300 may be used to determine efficacy of particular parameter settings of the IMD by testing parameter settings and recording patient feedback for each parameter setting. Information resulting from the testing may be presented to a user via the user interface 356. Programmer 300 may receive user input via the user interface device following generation of the output based on the first received information and the second received information, selecting one or more stimulation parameters for the delivery of the electrical stimulation. In one or more examples, the programmer 300 may generate a third set of stimulation parameters for delivery of the electrical stimulation based on the user input. In some examples, the programmer 300 compares the first information that was received relating to the first patient feedback with the second information relating to the second patient feedback, and automatically generates a third set of stimulation parameters for delivery of the electrical stimulation based on the comparison.

In an example, programmer 300 may be used to cause the IMD to automatically scan though a plurality of electrode combinations or parameter combinations. Processing circuitry 352 causes the IMD to automatically scan through each of a plurality of parameter combinations, including electrode combinations and parameter combinations. For each combination, the programmer 300 obtains and records the corresponding patient feedback.

Alternative to or in addition to the automatic scanning process, the user could manually advance scanning through electrode pairs and/or parameter combinations, for example with an arrow button on user interface 356. In some examples, as the user scans through the electrode pairs or parameter combinations to test and record patient feedback for each combination, the user may collect information such as a patient pain score indicating the degree of pain relief information from the combination, or a stimulation perception score indicating whether the patient perceives the stimulation, e.g., by verbal interaction with the patient or patient entry of information via a user input device, and enter the pain information into programmer 300 via user interface 356 of the programmer or the user input device.

Processing circuitry 352 controls stimulation circuitry 202 to deliver stimulation energy with stimulation parameters specified by one or more stimulation parameter settings 366 stored on storage device 354, and to collect patient feedback information pertaining to the stored stimulation parameter settings 366. Processing circuitry 352 may also control stimulation circuitry 202 to test different parameter settings and record corresponding patient feedback for each selected combination, and test different parameter settings as they compare to patient feedback. For example, processing circuitry 352 directs stimulation circuitry 202 to deliver stimulation with a particular cycling and patient feedback is collected from telemetry circuitry 358. The patient feedback data 364 for this test may be stored in the storage device 354.

Processing circuitry 352 may be configured to shift the previously tested cycling to a different cycling and collect the corresponding patient feedback. The patient feedback received for the stimulation at the changed stimulation parameter, in this example cycling, would be saved in the storage device 354. The processing circuitry 352 may continue to shift the cycling by either increasing or decreasing the cycling (e.g., the cycling frequency and/or cycling duty cycle), and record the respective patient feedback, which are stored on the storage device 354 and the information is output, e.g., via user interface 356. While the example of cycling is provided, processing circuitry 352 may direct stimulation circuitry to step through various incremental settings of other stimulation parameters, such as stimulation amplitude, stimulation pulse width, or stimulation frequency, and record the respective patient feedback information for each stepped value. Stimulation circuitry 202 may shift more than one stimulation parameter for each test and collect patient feedback information for the multiple shifted stimulation parameters.

In some examples, the processing circuitry 352 of programmer 300 directs delivery of electrical stimulation of the electrodes 232A, 232B, and receives information relating to patient feedback, and controls the delivery of electrical stimulation of the electrodes 232A, 232B based on the received patient feedback information in a closed loop setting. The patient feedback information may be received via the telemetry circuitry 358 either directly or indirectly from sensor 160 (FIG. 1) and/or a patient-input device.

The architecture of external programmer 300 illustrated in FIG. 3 is shown as an example. The techniques as set forth in this disclosure may be implemented in the example external programmer 300 of FIG. 3, as well as other types of systems not described specifically herein. Nothing in this disclosure should be construed so as to limit the techniques of this disclosure to the example architecture illustrated by FIG. 3.

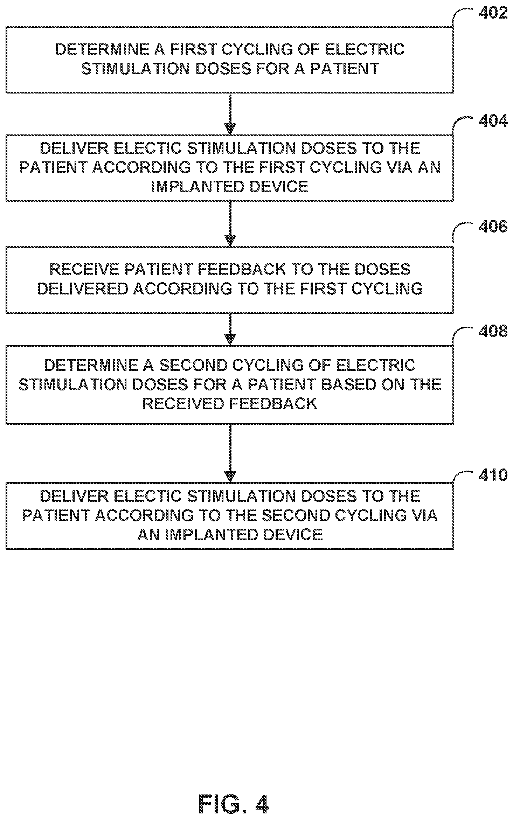

FIG. 4 is a flow diagram illustrating an example method of titrating a therapy, in accordance with one or more techniques of this disclosure. Although FIG. 4 is discussed using IMD 200A of FIG. 2A and external programmer 300 of FIG. 3, it is to be understood that the methods discussed herein may include and/or utilize other systems and methods in other examples.

A physician or clinician, IMD 200A, or external programmer 300 may determine a first cycling of electric stimulation doses for patient (402). For example, a physician or clinician may select values for a number of programmable stimulation parameters in order to define the electrical stimulation therapy to be delivered by the IMD 200A to the patient and may input the parameters as stimulation parameter settings 242 and/or 366, e.g., via user interface 356 of external programmer 300. The selected values for the stimulation parameters may include a first cycling of electric stimulation doses. In some examples, processing circuitry 210A, 210B, and/or 352 may determine and/or select the values for a number or programmable stimulation parameters, including a first cycling, according to executable instructions and, for example, based on information from sensors 222 in response to previous electric stimulation delivered to the patient.

IMD 200A may deliver one or more electric stimulation doses to the patient according to the determined first cycling (404). For example, processing circuitry 210A may control stimulation circuitry 202 to deliver stimulation energy via electrodes 232A, 232B with stimulation parameters specified by one or more stimulation parameter settings 242 stored on storage device, such as the determined first cycling.

IMD 200A may receive patient feedback representing a response of the patient to the one or more electric stimulation doses delivered according to the first cycling (406). For example, processing circuitry 210A may control stimulation circuitry 202, telemetry circuitry, and/or sensors 222 to collect patient feedback information, e.g., patient feedback data 254, by receiving the information via telemetry from a remote patient feedback sensor and/or patient-input device at a remote site. Processing circuitry 210A may store received patient feedback data 254 in storage device 212A. On some examples, IMD 200A may receive patient feedback as one or more of physiological signals, patient input, patient posture data, or any other suitable patient feedback type, signal, input, and the like. For example, IMD 200A may receive one or more ECAP and/or ECAP signals, LFPs, a heart rate, a heart rate variability, blood flow, a galvanic skin response, a network excitability, a signal and/or information related to a circadian rhythm, and the like. In some examples, IMD 200A may receive a pain response, a pain score, an area of pain, an amount of paresthesia, an area of paresthesia, a signal and/or information relating to voiding and/or a voiding rate (e.g., voids per day), and the like. In some examples, IMD 200A may receive patient posture and/or patient behavior data such as patient position, patient movement, patient movement history over a predetermined amount of time, a history of patent-selected stimulation parameters over a predetermined amount of time, and the like.

IMD 200A may determine, based on the patient feedback, a second cycling of the electric stimulation doses (408). In some examples, IMD 200A may determine, based on the feedback, that the patient does not need electric stimulation doses as often (e.g., relating to electric stimulation cycling rate or frequency or period) or for as long for each dose (e.g., relating to electric stimulation on-time, off-time, or cycling duty cycle). For example, the patient may be responding well to the therapy and doesn't need as much, or the patient may change position and/or posture in which the amount of electric stimulation may be reduced at least of a period of time, e.g., such as the patient is lying down or sleeping (or such as the patient waking up and/or moving around more). IMD 200A may determine a second cycling of the electric stimulation doses with a reduced cycling frequency and/or a reduced electric stimulation on-time (or an increased off-time) for each cycle. In other words, IMD 200A may determine a second cycling of the electric stimulation doses where an amount of on-time of each cycle of the second cycling is less than the amount of on-time of each cycle of the first cycling and/or where the cycling frequency of the second cycling is less than the cycling frequency of the first cycling. In some examples, IMD 200A may determine a second cycling that consumes less power of IMD 200A than the first cycling. In some examples, IMD 200A may determine the second cycling as part of an electric stimulation titration tapering process, e.g., via decreasing the electric stimulation dose via one of reduced cycling and/or reducing other stimulation parameter settings 242, namely, the electrode combination, pulse amplitude, pulse width, and/or pulse frequency of the stimulation doses.

In some examples, IMD 200A may determine, based on the patient feedback, an intermediate cycling of the electric stimulation doses. For example, IMD 200A may determine, based on the feedback, that the patient is not responding well to the first cycling, or may otherwise benefit from increasing electric stimulation dosing, e.g., before titrating the electric stimulation via the second cycling. In some examples, the patient may change position and/or posture in which the amount of electric stimulation may need to be increased for at least of a period of time, e.g., such as the patient is standing or moving (or that the patient is lying down or sleeping). IMD 200A may determine an intermediate cycling of the electric stimulation doses with an increased cycling frequency and/or an increased electric stimulation on-time (or a decreased off-time) for each cycle. In other words, IMD 200A may determine an intermediate cycling of the electric stimulation doses where an amount of on-time of each cycle of the intermediate cycling is greater than the amount of on-time of each cycle of the first cycling or the second cycling and/or where the cycling frequency of the intermediate cycling is greater than the cycling frequency of the first cycling or the second cycling. In some examples, IMD 200A may determine the intermediate cycling as part of an electric stimulation titration tapering process, e.g., via increasing the electric stimulation via one of reduced cycling and/or reducing other stimulation parameter settings 242, namely, the electrode combination, pulse amplitude, pulse width, and/or pulse frequency of the stimulation doses, subsequent to the first cycling and before decreasing the electric stimulation dose, e.g., via the second cycling with a reduced one or more of stimulation parameter settings 242.

IMD 200A may deliver one or more electric stimulation doses to the patient according the determined second cycling (410). For example, processing circuitry 210A may control stimulation circuitry 202 to deliver stimulation energy via electrodes 232A, 232B with stimulation parameters specified by one or more stimulation parameter settings 242 stored on storage device, such as the determined second cycling. In some examples, IMD 200A may deliver one or more electric stimulation doses according to a second cycling that consumes less power of IMD 200A than the first cycling.

In some examples, IMD 200A may receive patient feedback representing a response of the patient to the one or more electric stimulation doses delivered according to the second cycling, e.g., further patient feedback after titration via delivery of the second cycling. For example, the patient may start to do worse, or the patient posture may change such as waking up and moving around (or lying down or sleeping) such that the patient may require more stimulation therapy. In some examples, processing circuitry 210A may control stimulation circuitry 202, telemetry circuitry, and/or sensors 222 to collect patient feedback information, e.g., patient feedback data 254, such as described above. IMD 200A may determine, based on the patient feedback, to deliver one or more stimulation doses to the patient according to the first cycling. For example, IMD 200A may determine to switch the stimulation therapy back to the first cycling, with or without switching stimulation parameter settings 242 to those associated with the first cycling. IMD 200A may then deliver one or more electric stimulation doses to the patient according the first cycling.

In some examples, IMD 200A may dynamically change the electric stimulation delivered to the patient via changing the cycling and/or any of stimulation parameter settings 242, e.g., any number of times. For example, IMD 200A may receive still further patient feedback representing a response of the patient to the one or more electric stimulation doses delivered according to the first cycling, determine to switch the stimulation therapy back to the second cycling, with or without switching stimulation parameter settings 242 to those associated with the second cycling, deliver one or more electric stimulation doses to the patient according the second cycling, further patient feedback representing a response of the patient to the one or more electric stimulation doses delivered according to the second cycling, determine to switch the stimulation therapy back to the first cycling, with or without switching stimulation parameter settings 242 to those associated with the first cycling, deliver one or more electric stimulation doses to the patient according the first cycling, and repeat any number of times. In some examples, IMD 200A may dynamically change the electric stimulation cycling and/or any of the stimulation parameters 242 based on patient feedback any number of times to any number of cyclings and/or stimulation parameter settings, e.g., from the first to the second cycling, to a third cycling, to a fourth cycling, back to the second cycling, to the third cycling, to a fifth cycling, back to the first cycling, etc. In some example, IMD 200A may determine and deliver an electric stimulation cycling based on patient feedback and adapt the cycling (e.g., frequency and duty cycle) and/or electric stimulation parameters 242 over a period of time, e.g., to taper the titration of the electric stimulation doses.

In some examples, IMD 200A may titrate and/or taper the titration of two or more stimulation programs, e.g., concurrently or in succession. For example, a first electric stimulation program may treat a first set of symptoms or conditions and a second program may treat a second set of symptoms or conditions. In some examples, IMD 200A may titrate and/or taper the titration first and second stimulation programs via titrating any of the first and second cyclings and stimulation parameter settings of each program.

For example, IMD 200A may titrate and/or taper a first electric stimulation program by determining and delivering a first cycling of first electric stimulation doses to a patient, receiving patient feedback, and determining and delivering a second cycling of first electric stimulation doses to a patient as described above according to steps 402-410. IMD 200A may also titrate and/or taper a second electric stimulation program by determining and delivering a third cycling of second electric stimulation doses to a patient, receiving patient feedback, and determining and delivering a fourth cycling of second electric stimulation doses to a patient as described above according to steps 402-410, as described below.

A physician or clinician, IMD 200A, or external programmer 300 may determine a third cycling of second electric stimulation doses for patient, e.g., for a second stimulation program where one or both of the cycling and/or one or more stimulation parameter settings 242 (e.g., second electric stimulation doses) are different from the first cycling, such as described above with reference to method step 402. As described above, a physician or clinician may select values for a number of programmable stimulation parameters in order to define the electrical stimulation therapy to be delivered by the IMD 200A to the patient and may input the parameters as stimulation parameter settings 242 and/or 366, e.g., via user interface 356 of external programmer 300, e.g., second electric stimulation doses, and which may be different from or may be the same as the stimulation parameter settings 242 and/or 366 of the determined first cycling of the first stimulation program. Additionally, in some examples, the selected values for the stimulation parameters may include a third cycling of second electric stimulation doses, and the third cycling may be different from or may be the same as the first cycling of first electric stimulation doses. In some examples, one or both of the first and second cycling of the first program may be different from, or may be the same, as one or both of the third and fourth cycling of the second program, and alternatively or additionally, any one or more of the stimulation parameter settings 242 of the first electric stimulation doses of the first program may be different from any one or more of the stimulation parameter settings 242 of the second electric stimulation doses of the second program. For example, one or more of an electrode combination, an amplitude, and a pulse width of the one or more first electric stimulation doses may be different from a respective electrode combination, an amplitude, and a pulse width of the one or more second electric stimulation doses.

In some examples, processing circuitry 210A, 210B, and/or 352 may determine and/or select the values for a number or programmable stimulation parameters, including a third cycling, according to executable instructions and, for example, based on information from sensors 222 in response to previous electric stimulation delivered to the patient.

IMD 200A may deliver one or more second electric stimulation doses to the patient according to the determined third cycling (404). For example, processing circuitry 210A may control stimulation circuitry 202 to deliver stimulation energy via electrodes 232A, 232B with stimulation parameters specified by one or more stimulation parameter settings 242 stored on storage device, such as the determined third cycling.

IMD 200A may receive patient feedback representing a response of the patient to the one or more second electric stimulation doses delivered according to the third cycling (406), e.g., similar to that described above for the second cycling. For example, processing circuitry 210A may control stimulation circuitry 202, telemetry circuitry, and/or sensors 222 to collect patient feedback information, e.g., patient feedback data 254, by receiving the information via telemetry from a remote patient feedback sensor and/or patient-input device at a remote site. Processing circuitry 210A may store received patient feedback data 254 in storage device 212. On some examples, IMD 200A may receive patient feedback as one or more of physiological signals, patient input, patient posture data, or any other suitable patient feedback type, signal, input, and the like. For example, IMD 200A may receive one or more ECAP and/or ECAP signals, LFPs, a heart rate, a heart rate variability, blood flow, a galvanic skin response, a network excitability, a signal and/or information related to a circadian rhythm, and the like. In some examples, IMD 200A may receive a pain response, a pain score, an area of pain, an amount of paresthesia, an area of paresthesia, a signal and/or information relating to voiding and/or a voiding rate (e.g., voids per day), and the like. In some examples, IMD 200A may receive patient posture and/or patient behavior data such as patient position, patient movement, patient movement history over a predetermined amount of time, a history of patent-selected stimulation parameters over a predetermined amount of time, and the like.

IMD 200A may determine, based on the patient feedback, a fourth cycling of the second electric stimulation doses (408). IMD 200A may determine a fourth cycling of the second electric stimulation doses with a reduced cycling frequency and/or a reduced electric stimulation on-time (or an increased off-time) for each cycle. In other words, IMD 200A may determine a fourth cycling of the second electric stimulation doses where an amount of on-time of each cycle of the fourth cycling is less than the amount of on-time of each cycle of the third cycling and/or where the cycling frequency of the fourth cycling is less than the cycling frequency of the third cycling. In some examples, IMD 200A may determine a fourth cycling that consumes less power of IMD 200A than the third cycling. In some examples, IMD 200A may determine the fourth cycling as part of an electric stimulation titration tapering process, e.g., via decreasing the electric stimulation dose via one of reduced cycling and/or reducing other stimulation parameter settings 242, namely, the electrode combination, pulse amplitude, pulse width, and/or pulse frequency of the stimulation doses.

IMD 200A may deliver one or more second electric stimulation doses to the patient according the determined fourth cycling (410). For example, processing circuitry 210A may control stimulation circuitry 202 to deliver stimulation energy via electrodes 232A, 232B with stimulation parameters specified by one or more stimulation parameter settings 242 stored on storage device, such as the determined fourth cycling. In some examples, IMD 200A may deliver one or more second electric stimulation doses according to a fourth cycling that consumes less power of IMD 200A than the first cycling.

In some examples, the first and second cycling frequency of the first electric stimulation doses may correspond to one of a voiding frequency or network excitability, and the third and fourth cycling frequency of the second electric stimulation doses may correspond to a circadian rhythm of the patient.

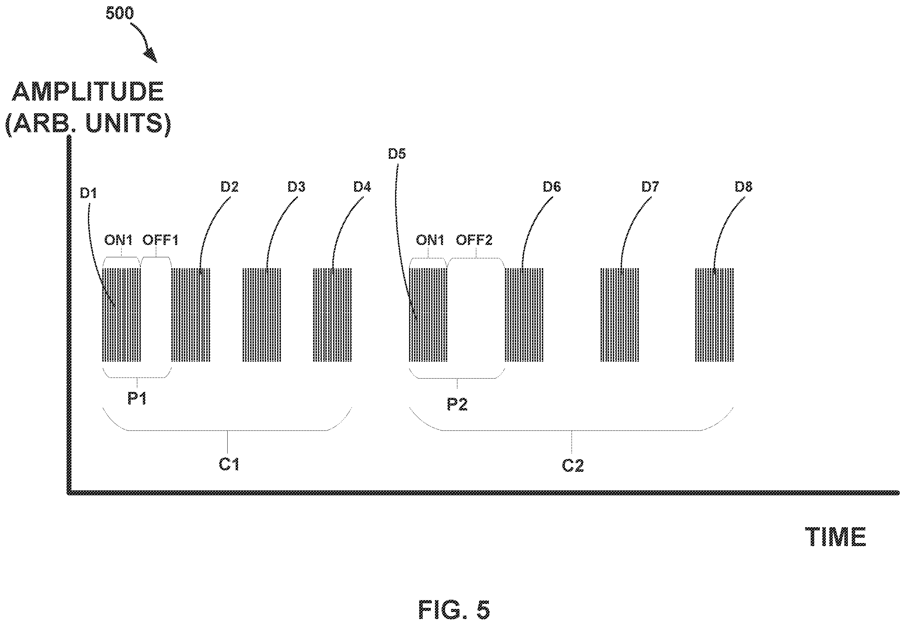

FIGS. 5-9 are a series of plots 500-900, respectively, illustrating examples of titrating electric stimulation doses, in accordance with one or more techniques of this disclosure. The series of plots illustrate examples of titrating electric stimulation doses via titrating stimulation cycle frequency, stimulation cycle duty cycle, both stimulation cycle frequency and duty cycle, and continuous titration tapering.

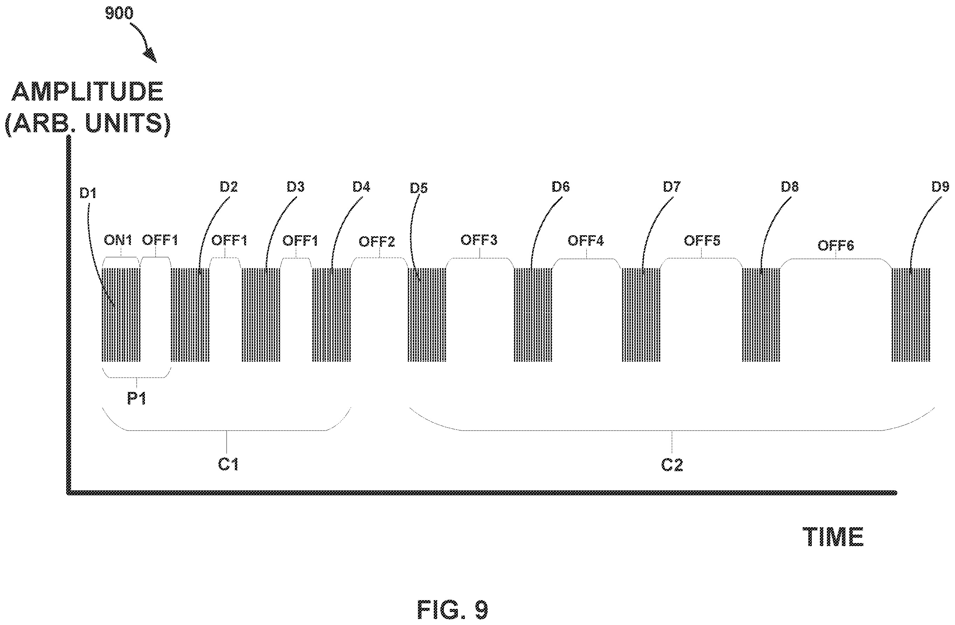

In the examples shown in each of FIGS. 5-9, each of doses D1-D8 (and D9 in FIG. 9) are substantially similar, e.g., each having a substantially similar electrode combination, amplitude, pulse frequency and pulse width. In some examples, each of doses D1-D9 may be different from each other, e.g., having different electrode combinations, amplitudes, pulse frequencies and pulse widths, or some may be substantially the same and some may be different. Although first cycling C1 and second cycling C2 each have four doses in FIGS. 5-8, it is to be understood that first cycling C1 and second cycling C2 may include any number of doses.

FIG. 5 is a plot 500 of an example of titrating electric stimulation doses, in accordance with one or more techniques of this disclosure. Plot 500 illustrates titrating electric stimulation doses via increasing the off-time between doses, resulting in reducing the frequency of the cycling as well. In the example shown, first cycling C1 includes substantially similar doses D1-D4 each having an on-time ON1 and an off-time OFF1. The frequency of first cycling C1 is the reciprocal of the cycle period P1 (e.g., 1/P1), and the duty cycle is the ratio ON1/OFF1. Second cycling C2 includes substantially similar doses D5-D8 each having an on-time ON1 and an off-time OFF2. The frequency of second cycling C2 is the reciprocal of the cycle period P2 (e.g., 1/P2), and the duty cycle is the ratio ON1/OFF2. In the example shown, the electric stimulation is titrated down via increasing the off-time, e.g., OFF2 is greater than OFF1. As a result, an IMD delivering electric stimulation doses according to cycling C2 consumes less power than delivering electric stimulation doses according to cycling C1, and the amount of on-time over a period of time is reduced, e.g., the amount of on-time for second cycling C2 is less than for first cycling C1 over a period of time, and the cycling frequency of second cycling C2 is less than first cycling C1. Additionally, the duty cycle of the second cycling is reduced by virtue of the increase in off-time, e.g., OFF2 > OFF1.

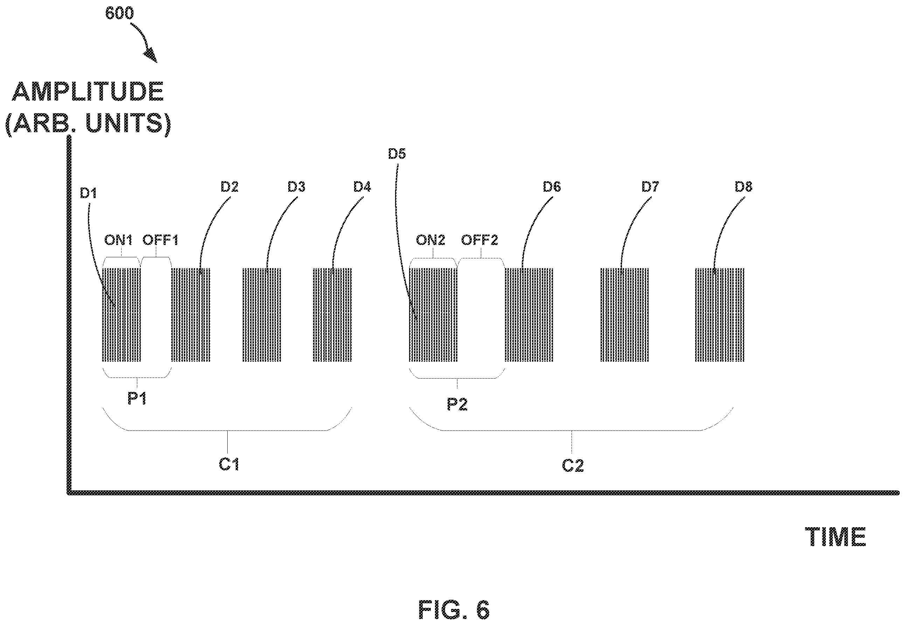

FIG. 6 is a plot 600 of an example of titrating electric stimulation doses, in accordance with one or more techniques of this disclosure. Plot 600 illustrates titrating electric stimulation doses via reducing the dosing frequency, however, the on-time of doses D5-D8 are increased to keep the duty cycle of second cycling C2 the same as C1. For example, the ratio of ON1/OFF1 may be substantially the same as ON2/OFF2 in the example of FIG. 6. Although titrating electric stimulation according to the example of FIG. 6 may not necessarily reduce the stimulation and/or power consumed by an IMD delivering the stimulation over time, reducing the frequency of the stimulation may be a useful intermediate step that may allow a patient to acclimate to a reduction in the amount of stimulation therapy via further titration in the future and/or to reduce the likelihood of developing a tolerance to the therapy by reducing its frequency but not the time-average of dosing received by the patient.

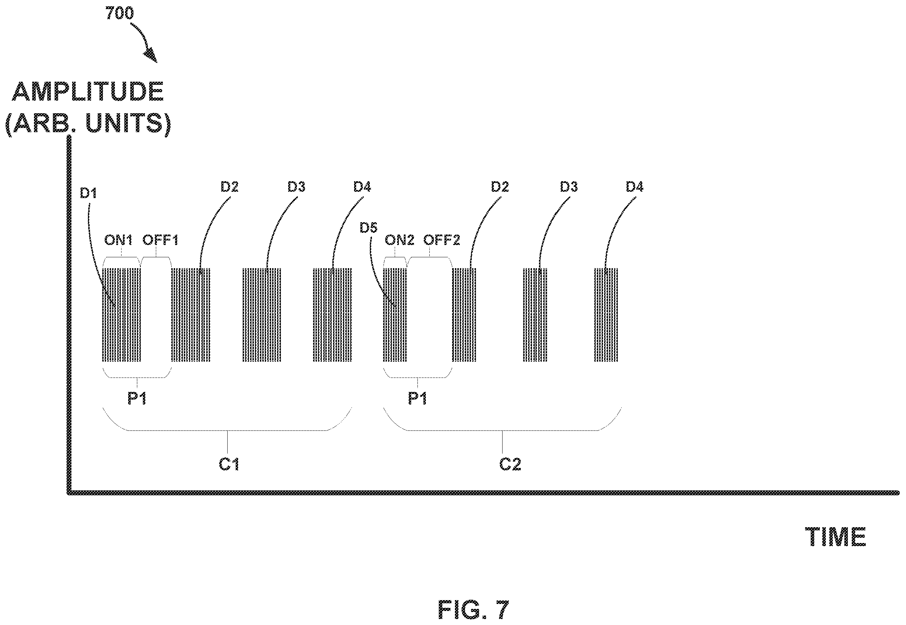

FIG. 7 is a plot 700 of an example of titrating electric stimulation doses, in accordance with one or more techniques of this disclosure. Plot 700 illustrates titrating electric stimulation doses via reducing the on-time of the doses. In the example shown, the frequency of each of first cycling C1 and second cycling C2 are kept the same by virtue of increasing the off-time by the same amount as the decrease in on-time, e.g., P1 = P2, however the on-time of second cycling C2 is reduced relative to first cycling C1, e.g., ON2 < ON1. Consequently, the duty cycle of the second cycling is also reduced, and an IMD delivering electric stimulation doses according to cycling C2 consumes less power than delivering electric stimulation doses according to cycling C1. Additionally, the amount of on-time, for each cycle as well as over a period of time, is reduced, e.g., the amount of on-time for second cycling C2 is less than for first cycling C1.

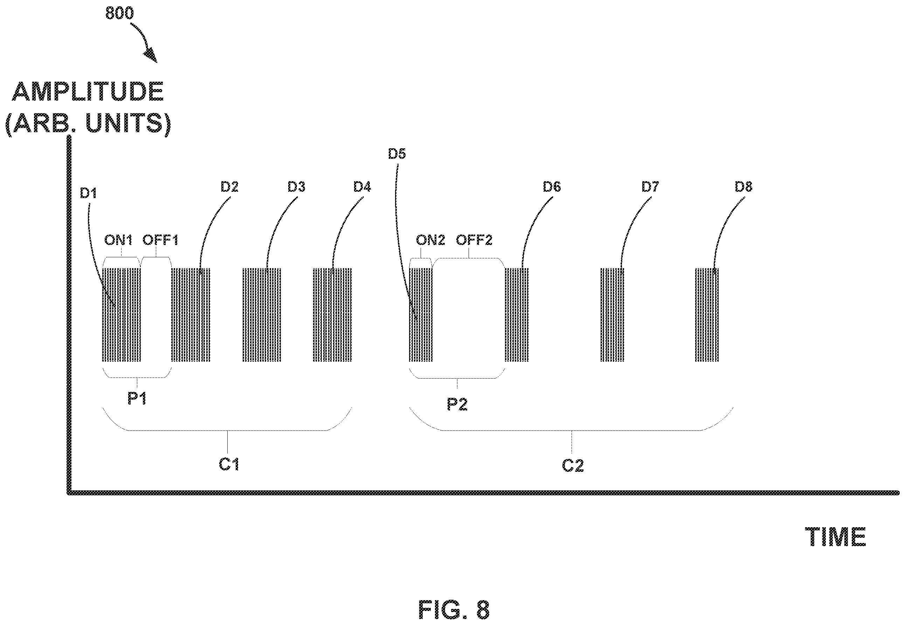

FIG. 8 is a plot 800 of an example of titrating electric stimulation doses, in accordance with one or more techniques of this disclosure. Plot 800 illustrates titrating electric stimulation doses via both increasing the off-time and reducing the on-time of the doses. Namely, ON2 < ON1, OFF2 > OFF1, and P2 > P1 (e.g., the frequency of the second cycling is reduced relative to the first cycling via an increase in the cycling period). As such, the duty cycle of the second cycling is reduced relative to the first cycling, and an IMD delivering electric stimulation doses according to cycling C2 consumes less power than delivering electric stimulation doses according to cycling C1.

FIG. 9 is a plot 900 of an example of titrating electric stimulation doses, in accordance with one or more techniques of this disclosure. Plot 900 illustrates titrating electric stimulation doses via a continuous taper of the electric stimulation doses by continuously increasing the off-time between the doses, e.g., OFF1 < OFF2 < OFF3 < OFF4 < OFF5 < OFF6. In the example shown, the first four does D1-D4 are delivered according to first cycling C1 with an on-time ON1, an off-time OFF1, a cycling frequency 1/P1, and a cycling duty cycle ON1/OFF1. In the example shown, the second cycling C2 does not have a constant period or off-time, but rather both increase between each of doses D5-D9. In the example shown, the on-times ON1 of all doses D1-D9 are substantially the same, however, it need not be so, and in some examples the on-time of any of doses D5-D9 may be increased and or decreased relative to ON1. In the example shown, the amount of electric stimulation delivered over the time period of second cycling C2 continuously decreases via the continuous increase in off-time and a continuous decrease in cycling frequency. In some examples, second cycling C2 may continuously decrease the on-time. In the example shown, an IMD delivering electric stimulation doses according to cycling C2 consumes less power than delivering electric stimulation doses according to cycling C1.

The techniques described in this disclosure may be implemented, at least in part, in hardware, software, firmware or any combination thereof For example, various aspects of the described techniques may be implemented within processing circuitry, which may include one or more processors, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry A control unit including hardware may also form one or more processors or processing circuitry configured to perform one or more of the techniques of this disclosure.

Such hardware, software, and firmware may be implemented, and various operation may be performed within same device, within separate devices, and/or on a coordinated basis within, among or across several devices, to support the various operations and functions described in this disclosure. In addition, any of the described units, circuits or components may be implemented together or separately as discrete but interoperable logic devices. Depiction of different features as circuits or units is intended to highlight different functional aspects and does not necessarily imply that such circuits or units must be realized by separate hardware or software components. Rather, functionality associated with one or more circuits or units may be performed by separate hardware or software components or integrated within common or separate hardware or software components. Processing circuitry described in this disclosure, including a processor or multiple processors, may be implemented, in various examples, as fixed-function circuits, programmable circuits, or a combination thereof. Fixed-function circuits refer to circuits that provide particular functionality with preset operations. Programmable circuits refer to circuits that can be programmed to perform various tasks and provide flexible functionality in the operations that can be performed. For instance, programmable circuits may execute software or firmware that cause the programmable circuits to operate in the manner defined by instructions of the software or firmware. Fixed-function circuits may execute software instructions (e.g., to receive stimulation parameters or output stimulation parameters), but the types of operations that the fixed-function circuits perform are generally immutable. In some examples, one or more of the units may be distinct circuit blocks (fixed-function or programmable), and in some examples, one or more of the units may be integrated circuits.

The techniques described in this disclosure may also be embodied or encoded in a computer-readable medium, such as a computer-readable storage medium, containing instructions that may be described as non-transitory media. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

## Claims

1. A system comprising:
electrical stimulation circuitry (202) configured to generate electrical stimulation including electric stimulation doses, the electrical stimulation comprising a cycling including a cycling frequency and a cycling duty cycle, wherein the cycling duty cycle is a ratio between an amount of on-time and an amount of off-time of the electric stimulation doses and the cycling frequency is a rate at which electric stimulation doses are delivered;
electrodes configured to deliver the electrical stimulation to a patient; and
processing circuitry (210A, 210B) configured to:
determine, for a patient, a first cycling of electric stimulation doses;
deliver the electric stimulation doses according to the determined first cycling;
receive patient feedback representing a response of the patient to the electric stimulation doses delivered according to the first cycling;
determine, based on the patient feedback, a second cycling of the electric stimulation doses; and
deliver the electric stimulation doses to the patient according to the determined second cycling, wherein delivering the electric stimulation doses for the patient according to the determined second cycling consumes less power of the implanted device than delivering the electric stimulation doses for the patient according to the determined first cycling.

2. The system of claim 1, wherein the patient feedback comprises one or more of a physiological signal, a patient input, or patient posture data.

3. The system of claim 2, wherein the physiological signal comprises one of an evoked compound action potential (ECAP), a local field potential (LFP), a heart rate, a heart rate variability, a blood flow, a galvanic skin response, or a network excitability.

4. The system of any one of claims 2-3, wherein the patient input comprises one of a pain response, a pain score, an area of pain, an amount of paresthesia, an area of paresthesia, or a voiding rate.

5. The system of any one of claims 2-4, wherein the patient posture data comprises one of a patient position, a patient movement, a patient movement history over a predetermined amount of time, a history of patent-selected stimulation parameters over a predetermined amount of time.

6. The system of any one of claims 1-5, wherein an amount of on-time of each cycle of the second cycling is less than the amount of on-time of each cycle of the first cycling.

7. The system of any one of claims 1-6, wherein the cycling frequency of the second cycling is less than the cycling frequency of the first cycling.

8. The system of any one of claims 1-7, wherein one of the amplitude, pulse width, or pulse frequency of the electric stimulation doses of the second cycling is less than the amplitude, pulse width, or pulse frequency of the electric stimulation doses of the first cycling.

9. The system of any one of claims 1-8, wherein the processing circuitry (210A, 210B) is further configured to:
determine, based on the patient feedback, an intermediate cycling of the electric stimulation doses; and
deliver, the electric stimulation doses to the patient according to the determined intermediate cycling,
wherein at least one of the amount of on-time of each cycle of the intermediate cycling is greater than an amount of on-time of each cycle of the first cycling, a cycling frequency of the intermediate cycling is greater than a cycling frequency of the first cycling, or an amplitude, pulse width, or pulse frequency of the electric stimulation doses of the intermediate cycling is greater than an amplitude, pulse width, or the first pulse frequency of the electric stimulation doses of the first cycling.

10. The system of any one of claims 1-9, wherein the processing circuitry (210A, 210B) is further configured to:
receive patient feedback representing a response of the patient to the electric stimulation doses according to the second cycling;
determine, based on the patient feedback representing the response, to deliver the electric stimulation doses to the patient according to the first cycling; and
deliver the electric stimulation doses to the patient according the first cycling.

11. The system of any one of claims 1-10, wherein the electric stimulation doses comprise first electric stimulation doses, wherein the processing circuitry (210A, 210B) is further configured to:
determine, for the patient, a third cycling of second electric stimulation doses;
deliver second electric stimulation doses to the patient according the determined third cycling;
receive patient feedback representing a response of the patient to the second electric stimulation doses delivered according to the third cycling;
determine, based on the patient feedback, a fourth cycling of the second electric stimulation doses; and
deliver the second electric stimulation doses to the patient according the determined fourth cycling, wherein delivering the second electric stimulation doses for the patient according to the determined fourth cycling consumes less power of the implanted device than delivering the second electric stimulation doses for the patient according to the determined third cycling.

12. The system of claim 11, wherein one or more of an electrode combination, an amplitude, and a pulse width of the first electric stimulation doses is different from a respective electrode combination, an amplitude, and a pulse width of the second electric stimulation doses.

13. The system of any one of claims 10-12, wherein the first and third cycling are the same, wherein the second and fourth cycling are the same.

14. The system of any one of claims claim 10-13, wherein the first and second cycling frequency of the first electric stimulation doses corresponds to one of a voiding frequency or network excitability, wherein the third and fourth cycling frequency of the second electric stimulation doses corresponds to a circadian rhythm of the patient.

15. A computer readable medium comprising instructions that when executed cause one or more processors to:
determine, for a patient, a first cycling of electric stimulation doses, the first cycling including a cycling frequency and a cycling duty cycle, wherein the cycling duty cycle is a ratio between an amount of on-time and an amount of off-time of the electric stimulation doses and the cycling frequency is a rate at which electric stimulation doses are delivered;
deliver the electric stimulation doses according to the determined first cycling;
receive patient feedback representing a response of the patient to the electric stimulation doses delivered according to the first cycling;
determine, based on the patient feedback, a second cycling of the electric stimulation doses, the second cycling including a cycling frequency and a cycling duty cycle, wherein the cycling duty cycle is a ratio between an amount of on-time and an amount of off-time of the electric stimulation doses and the cycling frequency is a rate at which electric stimulation doses are delivered; and
deliver the electric stimulation doses to the patient according to the determined second cycling, wherein delivering the electric stimulation doses for the patient according to the determined second cycling consumes less power of the implanted device than delivering the electric stimulation doses for the patient according to the determined first cycling,
wherein one or both of an amount of on-time of each cycle or a frequency of the second cycling is less than one or both of a corresponding amount of on-time of each cycle or a corresponding frequency of the first cycling,
wherein the patient feedback comprises one or more of a physiological signal, a patient input, patient posture data, an evoked compound action potential (ECAP), a local field potential (LFP), a heart rate, a heart rate variability, a blood flow, a galvanic skin response, a network excitability, a pain response, a pain score, an area of pain, an amount of paresthesia, an area of paresthesia, or a voiding rate.

## Patentansprüche

1. System, umfassend:
eine elektrische Stimulationsschaltung (202), die dazu ausgelegt ist, eine elektrische Stimulation mit Stimulationsdosen zu erzeugen, wobei die elektrische Stimulation einen Zyklus mit einer Zyklusfrequenz und einem Zyklustastgrad umfasst, wobei der Zyklustastgrad ein Verhältnis zwischen einer Dauer der Ein-Zeit und einer Dauer der Aus-Zeit der elektrischen Stimulationsdosen ist und die Zyklusfrequenz eine Rate ist, mit der die elektrischen Stimulationsdosen abgegeben werden:
Elektroden, die dazu ausgelegt sind, die elektrische Stimulation an einen Patienten abzugeben; und
eine Verarbeitungsschaltung (210A, 210B), ausgelegt zum: Bestimmen eines ersten Zyklus elektrischer Stimulationsdosen für einen Patienten;
Abgeben der elektrischen Stimulationsdosen entsprechend dem bestimmten ersten Zyklus;
Empfangen einer Patientenrückmeldung, die eine Antwort des Patienten auf die elektrischen Stimulationsdosen darstellt, die entsprechend dem ersten Zyklus abgegeben wurden;
Bestimmen eines zweiten Zyklus der elektrischen Stimulationsdosen basierend auf der Patientenrückmeldung; und
Abgeben der elektrischen Stimulationsdosen an den Patienten entsprechend dem bestimmten zweiten Zyklus, wobei das Abgeben der elektrischen Stimulationsdosen an den Patienten entsprechend dem bestimmten zweiten Zyklus weniger Leistung der implantierten Vorrichtung verbraucht als das Abgeben der elektrischen Stimulationsdosen an den Patienten entsprechend dem bestimmten ersten Zyklus.

2. System nach Anspruch 1, wobei die Patientenrückmeldung ein physiologisches Signal, eine Patienteneingabe und/oder Patientenhaltungsdaten umfasst.

3. System nach Anspruch 2, wobei das physiologische Signal ein evoziertes zusammengesetztes Aktionspotential (ECAP), ein lokales Feldpotential (LFP), eine Herzfrequenz, eine Herzfrequenzvariabilität, einen Blutfluss, eine galvanische Hautantwort oder eine Netzwerk-Erregbarkeit umfasst.

4. System nach einem der Ansprüche 2 bis 3, wobei die Patienteneingabe eine Schmerzantwort, einen Schmerz-Score, einen Schmerzbereich, ein Ausmaß an Parästhesie, einen Parästhesiebereich oder eine Entleerungsrate umfasst.

5. System nach einem der Ansprüche 2 bis 4, wobei die Patientenhaltungsdaten eine Patientenposition, eine Patientenbewegung, einen Patientenbewegungsverlauf über eine vorbestimmte Zeitdauer oder einen Verlauf der vom Patienten ausgewählten Stimulationsparameter über eine vorbestimmte Zeitdauer umfasst.

6. System nach einem der Ansprüche 1 bis 5, wobei eine Dauer der Ein-Zeit jedes Zyklus des zweiten Zyklus geringer ist als die Dauer der Ein-Zeit jedes Zyklus des ersten Zyklus.

7. System nach einem der Ansprüche 1 bis 6, wobei die Zyklusfrequenz des zweiten Zyklus geringer ist als die Zyklusfrequenz des ersten Zyklus.

8. System nach einem der Ansprüche 1 bis 7, wobei die Amplitude, die Impulsbreite oder die Impulsfrequenz der elektrischen Stimulationsdosen des zweiten Zyklus geringer ist als die Amplitude, Impulsbreite oder Impulsfrequenz der elektrischen Stimulationsdosen des ersten Zyklus.

9. System nach einem der Ansprüche 1 bis 8, wobei die Verarbeitungsschaltung ferner (210A, 210B) ausgelegt ist zum:
Bestimmen eines Zwischenzyklus der elektrischen Stimulationsdosen basierend auf der Patientenrückmeldung; und
Abgeben der elektrischen Stimulationsdosen an den Patienten entsprechend dem bestimmten Zwischenzyklus;
wobei die Dauer der Ein-Zeit jedes Zyklus des Zwischenzyklus größer ist als die Dauer der Ein-Zeit jedes Zyklus des ersten Zyklus und/oder eine Zyklusfrequenz des Zwischenzyklus größer ist als eine Zyklusfrequenz des ersten Zyklus und/oder eine Amplitude, Impulsbreite oder Impulsfrequenz der elektrischen Stimulationsdosen des Zwischenzyklus größer ist als eine Amplitude, Impulsbreite oder die erste Impulsfrequenz der elektrischen Stimulationsdosen des ersten Zyklus.

10. System nach einem der Ansprüche 1 bis 9, wobei die Verarbeitungsschaltung (210A, 210B) ferner ausgelegt ist zum:
Empfangen einer Patientenrückmeldung, die eine Antwort des Patienten auf die elektrischen Stimulationsdosen darstellt, die entsprechend dem zweiten Zyklus abgegeben wurden;
Bestimmen, die elektrischen Stimulationsdosen an den Patienten entsprechend dem ersten Zyklus abzugeben, basierend auf der Patientenrückmeldung, die die Antwort darstellt; und
Abgeben der elektrischen Stimulationsdosen an den Patienten entsprechend dem ersten Zyklus;

11. System nach einem der Ansprüche 1 bis 10. wobei die elektrischen Stimulationsdosen erste elektrische Stimulationsdosen umfassen, wobei die Verarbeitungsschaltung (210A, 210B) ferner ausgelegt ist zum:
Bestimmen eines dritten Zyklus zweiter elektrischer Stimulationsdosen für einen Patienten;
Abgeben zweiter elektrischer Stimulationsdosen an den Patienten entsprechend dem bestimmten dritten Zyklus;
Empfangen einer Patientenrückmeldung, die eine Antwort des Patienten auf die zweiten elektrischen Stimulationsdosen darstellt, die entsprechend dem dritten Zyklus abgegeben wurden;
Bestimmen eines vierten Zyklus der zweiten elektrischen Stimulationsdosen basierend auf der Patientenrückmeldung; und
Abgeben der zweiten elektrischen Stimulationsdosen an den Patienten entsprechend dem bestimmten vierten Zyklus, wobei das Abgeben der zweiten elektrischen Stimulationsdosen an den Patienten entsprechend dem bestimmten vierten Zyklus weniger Leistung der implantierten Vorrichtung verbraucht als das Abgeben der zweiten elektrischen Stimulationsdosen an den Patienten entsprechend dem bestimmten dritten Zyklus.

12. System nach Anspruch 11, wobei eine Elektrodenkombination, eine Amplitude und/oder eine Impulsbreite der ersten elektrischen Stimulationsdosen von einer entsprechenden Elektrodenkombination, einer Amplitude und einer Impulsbreite der zweiten elektronischen Stimulationsdosen verschieden ist.

13. System nach einem der Ansprüche 10 bis 12, wobei der erste und dritte Zyklus gleich sind, wobei der zweite und vierte Zyklus gleich sind.

14. System nach einem der Ansprüche 10 bis 13, wobei die erste und zweite Zyklusfrequenz der ersten elektrischen Stimulationsdosen einer Entleerungsfrequenz oder einer Netzwerk-Erregbarkeit entsprechen, wobei die dritte und vierte Zyklusfrequenz der zweiten elektrischen Stimulationsdosen einem zirkakadianen Rhythmus des Patienten entsprechen.

15. Computerlesbares Medium, das Anweisungen umfasst, die bei Ausführung bewirken, dass der Prozessor Folgendes ausführt:
Bestimmen eines ersten Zyklus elektrischer Stimulationsdosen für einen Patienten, wobei der erste Zyklus eine Zyklusfrequenz und einen Zyklustastgrad aufweist, wobei der Zyklustastgrad ein Verhältnis zwischen einer Dauer der Ein-Zeit und einer Dauer der Aus-Zeit der elektrischen Stimulationsdosen ist und die Zyklusfrequenz eine Rate ist, mit der die elektrischen Stimulationsdosen abgegeben werden;
Abgeben der elektrischen Stimulationsdosen entsprechend dem bestimmten ersten Zyklus:
Empfangen einer Patientenrückmeldung, die eine Antwort des Patienten auf die elektrischen Stimulationsdosen darstellt, die entsprechend dem ersten Zyklus abgegeben wurden;
Bestimmen eines zweiten Zyklus der elektrischen Stimulationsdosen basierend auf der Patientenrückmeldung, wobei der zweite Zyklus eine Zyklusfrequenz und einen Zyklustastgrad aufweist, wobei der Zyklustastgrad ein Verhältnis zwischen einer Dauer der Ein-Zeit und einer Dauer der Aus-Zeit der elektrischen Stimulationsdosen ist und die Zyklusfrequenz eine Rate ist, mit der die elektrischen Stimulationsdosen abgegeben werden; und
Abgeben der elektrischen Stimulationsdosen an den Patienten entsprechend dem bestimmten zweiten Zyklus, wobei das Abgeben der elektrischen Stimulationsdosen an den Patienten entsprechend dem bestimmten zweiten Zyklus weniger Leistung der implantierten Vorrichtung verbraucht als das Abgeben der elektrischen Stimulationsdosen an den Patienten entsprechend dem bestimmten ersten Zyklus,
wobei eine Dauer der Ein-Zeit jedes Zyklus und/oder eine Frequenz des zweiten Zyklus geringer ist als eine entsprechende Dauer der Ein-Zeit jedes Zyklus und/oder eine entsprechende Frequenz des ersten Zyklus,
wobei die Patientenrückmeldung ein physiologisches Signal, eine Patienteneingabe, Patientenhaltungsdaten, ein evoziertes zusammengesetztes Aktionspotential (ECAP), ein lokales Feldpotential (LFP), eine Herzfrequenz, eine Herzfrequenzvariabilität, einen Blutfluss, eine galvanische Hautantwort, eine Netzwerk-Erregbarkeit, eine Schmerzantwort, einen Schmerzbereich, ein Ausmaß an Parästhesie, einen Parästhesiebereich und/oder eine Entleerungsrate umfasst.

## Revendications

1. Système comprenant :
des circuits de stimulation électrique (202) configurés pour générer une stimulation électrique comprenant des doses de stimulation électrique, la stimulation électrique comprenant un cycle comprenant une fréquence de cycle et un cycle de service, le cycle de service étant un rapport entre une quantité de temps d'activation et une quantité de temps d'arrêt des doses de stimulation électrique et la fréquence de cycle étant une vitesse à laquelle des doses de stimulation électrique sont administrées :
des électrodes configurées pour administrer la stimulation électrique à un patient ; et
des circuits de traitement (210A, 210B) configurés pour : déterminer pour un patient, un premier cycle de doses de stimulation électrique,
administrer les doses de stimulation électrique selon le premier cycle déterminé ;
recevoir une rétroaction de patient représentant une réponse du patient aux doses de stimulation électrique administrées selon le premier cycle ;
déterminer, sur la base de la rétroaction de patient, un deuxième cycle des doses de stimulation électrique ; et
administrer les doses de stimulation électrique au patient selon le deuxième cycle déterminé, l'administration des doses de stimulation électrique au patient selon le deuxième cycle déterminé consommant moins d'énergie du dispositif implanté que l'administration des doses de stimulation électrique au patient selon le premier cycle déterminé.

2. Système selon la revendication 1, la rétroaction de patient comprenant un ou plusieurs d'un signal physiologique, d'une entrée de patient ou de données de posture de patient.

3. Système selon la revendication 2, le signal physiologique comprenant un élément parmi un potentiel d'action de composé évoqué (ECAP), un potentiel de champ local (LFP), une fréquence cardiaque, une variabilité de fréquence cardiaque, un débit sanguin, une réponse cutanée galvanique ou une excitabilité de réseau.

4. Système selon l'une quelconque des revendications 2 et 3, l'entrée de patient comprenant un élément parmi une réponse à la douleur, un score de douleur, une zone de douleur, une quantité de paresthésie, une zone de paresthésie ou un taux de miction.

5. Système selon l'une quelconque des revendications 2 à 4, les données de posture de patient comprenant un élément parmi une position de patient, un mouvement de patient, un historique des mouvements de patient sur une quantité de temps prédéterminée, un historique des paramètres de stimulation sélectionnés par le patient sur une quantité de temps prédéterminée.

6. Système selon l'une quelconque des revendications 1 à 5, une quantité de temps d'activation de chaque cycle du deuxième cycle étant inférieure à la quantité de temps d'activation de chaque cycle du premier cycle.

7. Système selon l'une quelconque des revendications 1 à 6, la fréquence de cycle du deuxième cycle étant inférieure à la fréquence de cycle du premier cycle.

8. Système selon l'une quelconque des revendications 1 à 7, l'un parmi l'amplitude, la largeur d'impulsion ou la fréquence d'impulsion des doses de stimulation électrique du deuxième cycle étant inférieure à l'amplitude, la largeur d'impulsion ou la fréquence d'impulsion des doses de stimulation électrique du premier cycle.

9. Système selon l'une quelconque des revendications 1 à 8, les circuits de traitement (210A, 210B) étant en outre configurés pour :
déterminer, sur la base de la rétroaction de patient, un cycle intermédiaire des doses de stimulation électrique ; et
administrer, les doses de stimulation électrique au patient selon le cycle intermédiaire déterminé,
au moins l'une parmi la quantité de temps d'activation de chaque cycle du cycle intermédiaire étant supérieure à une quantité de temps d'activation de chaque cycle du premier cycle, une fréquence de cycle du cycle intermédiaire étant supérieure à une fréquence de cycle du premier cycle, ou une amplitude, une largeur d'impulsion ou une fréquence d'impulsion des doses de stimulation électrique du cycle intermédiaire étant supérieure à une amplitude, une largeur d'impulsion, ou à la première fréquence d'impulsion des doses de stimulation électrique du premier cycle.

10. Système selon l'une quelconque des revendications 1 à 9, les circuits de traitement (210A, 210B) étant en outre configurés pour :
recevoir une rétroaction de patient représentant une réponse du patient aux doses de stimulation électrique selon le deuxième cycle ;
déterminer, sur la base de la rétroaction de patient représentant la réponse, d'administrer les doses de stimulation électrique au patient selon le premier cycle ; et
administrer les doses de stimulation électrique au patient selon le premier cycle.

11. Système selon l'une quelconque des revendications 1 à 10, les doses de stimulation électrique comprenant des premières doses de stimulation électrique, les circuits de traitement (210A, 210B) étant en outre configurés pour :
déterminer, pour le patient, un troisième cycle de deuxièmes doses de stimulation électrique ;
administrer des deuxièmes doses de stimulation électrique au patient selon le troisième cycle déterminé ;
recevoir une rétroaction de patient représentant une réponse du patient aux deuxièmes doses de stimulation électrique administrées selon le troisième cycle ;
déterminer, sur la base de la rétroaction de patient, un quatrième cycle des deuxièmes doses de stimulation électrique ; et
administrer les deuxièmes doses de stimulation électrique au patient selon le quatrième cycle déterminé, l'administration des deuxièmes doses de stimulation électrique au patient selon le quatrième cycle déterminé consommant moins d'énergie du dispositif implanté que l'administration des deuxièmes doses de stimulation électrique au patient selon le troisième cycle déterminé.

12. Système selon la revendication 11, une ou plusieurs d'une combinaison d'électrodes, d'une amplitude et d'une largeur d'impulsion des premières doses de stimulation électrique étant différentes d'une combinaison d'électrodes respective, d'une amplitude et d'une largeur d'impulsion des deuxièmes doses de stimulation électrique.

13. Système selon l'une quelconque des revendications 10 à 12, les premier et troisième cycles étant les mêmes, les deuxième et quatrième cycles étant les mêmes.

14. Système selon l'une quelconque des revendications 10 à 13, la première et la deuxième fréquence de cycle des premières doses de stimulation électrique correspondant à l'une d'une fréquence de miction ou d'une excitabilité de réseau, la troisième et la quatrième fréquence de cycle des deuxièmes doses de stimulation électrique correspondant à un rythme circadien du patient.

15. Support lisible par ordinateur comprenant des instructions qui, lorsqu'elles sont exécutées amènent un ou plusieurs processeurs à :
déterminer, pour un patient, un premier cycle de doses de stimulation électrique, le premier cycle comprenant une fréquence de cycle et un cycle de service, le cycle de service étant un rapport entre une quantité de temps d'activation et une quantité de temps d'arrêt des doses de stimulation électrique et la fréquence de cycle étant une vitesse à laquelle des doses de stimulation électrique sont administrées ;
administrer les doses de stimulation électrique selon le premier cycle déterminé :
recevoir une rétroaction de patient représentant une réponse du patient aux doses de stimulation électrique administrées selon le premier cycle ;
déterminer, sur la base de la rétroaction de patient, un deuxième cycle des doses de stimulation électrique, le deuxième cycle comprenant une fréquence de cycle et un cycle de service, le cycle de service étant un rapport entre une quantité de temps d'activation et une quantité de temps d'arrêt des doses de stimulation électrique et la fréquence de cycle étant une vitesse à laquelle des doses de stimulation électrique sont administrées ; et
administrer les doses de stimulation électrique au patient selon le deuxième cycle déterminé, l'administration des doses de stimulation électrique au patient selon le deuxième cycle déterminé consommant moins d'énergie du dispositif implanté que l'administration des doses de stimulation électrique au patient selon le premier cycle déterminé,
l'une ou les deux d'une quantité de temps d'activation de chaque cycle ou d'une fréquence du deuxième cycle étant inférieure à l'une ou aux deux d'une quantité de temps d'activation de chaque cycle correspondante ou d'une fréquence correspondante du premier cycle,
la rétroaction de patient comprenant un ou plusieurs éléments parmi un signal physiologique, une entrée de patient, des données de posture de patient, un potentiel d'action de composé évoqué (ECAP), un potentiel de champ local (LFP), une fréquence cardiaque, une variabilité de fréquence cardiaque, un débit sanguin, une réponse cutanée galvanique, une excitabilité de réseau, une réponse à la douleur, un score de douleur, une zone de douleur, une quantité de paresthésie, une zone de paresthésie ou un taux de miction.
